(19) Europäisches Patentamt
European Patent Office
Office européen des brevets

(11) Veröffentlichungsnummer: **0 322 361 A2**

(12) # EUROPÄISCHE PATENTANMELDUNG

(21) Anmeldenummer: **88810860.2**

(22) Anmeldetag: **14.12.88**

(51) Int. Cl.$^4$: **C 07 D 405/04**
C 07 D 405/06,
C 07 D 311/58,
C 07 D 311/70, A 61 K 31/445

(30) Priorität: **23.12.87 CH 5008/87**

(43) Veröffentlichungstag der Anmeldung:
**28.06.89 Patentblatt 89/26**

(84) Benannte Vertragsstaaten:
**AT BE CH DE ES FR GB GR IT LI LU NL SE**

(71) Anmelder: **CIBA-GEIGY AG**
**Klybeckstrasse 141**
**CH-4002 Basel (CH)**

(72) Erfinder: **Fröstl, Wolfgang, Dr.**
**St. Johanns-Vorstadt 6/2**
**CH-4056 Basel (CH)**

Patentansprüche für folgende Vertragsstaaten: ES + GR.

(54) **Weitere hydrierte 1-Benzooxacycloalkylpyridincarbonsäureverbindungen.**

(57) Die Erfindung betrifft neue hydrierte 1-Benzooxacycloalkylpyridincarbonsäureverbindungen der Formel

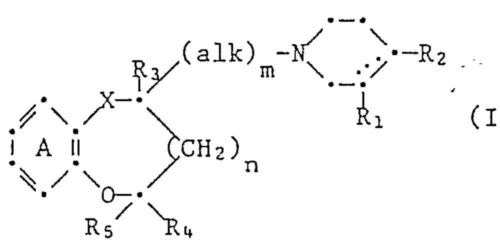

(I),

worin entweder $R_1$ Carboxy, Niederalkoxycarbonyl, amidiertes Carboxy oder gegebenenfalls acyliertes Hydroxymethyl bedeutet und $R_2$ Wasserstoff, eine gegebenenfalls verätherte oder acylierte Hydroxygruppe oder eine gegebenenfalls acylierte Aminogruppe darstellt oder $R_1$ Wasserstoff bedeutet und $R_2$ für Carboxy, Niederalkoxycarbonyl, amidiertes Carboxy oder gegebenenfalls acyliertes Hydroxymethyl steht und worin $R_3$ Wasserstoff oder Niederalkyl bedeutet, $R_4$ Niederalkyl ist, $R_5$ Niederalkyl darstellt, alk Niederalkylen oder Niederalkyliden bedeutet, der Ring A unsubstituiert oder ein- oder mehrfach durch Hydroxy, Niederalkoxy, Niederalkanoyloxy, Cyano, Halogen, Niederalkyl und/oder Trifluormethyl substituiert ist, die gestrichelte Linie zum Ausdruck bringen soll, dass zwischen den Kohlenstoffatomen, welche die Substituenten $R_1$ und $R_2$ tragen, eine Einfach-oder eine Doppelbindung vorliegt, m 0 oder 1 bedeutet und worin entweder X ein Sauerstoffatom oder eine Methylengruppe bedeutet und n für 0 steht oder X für eine direkte Bindung steht und n für 1 steht, und ihre Tautomeren und/oder Salze. Diese Verbindungen können als pharmazeutische Wirkstoffe verwendet werden und sind in an sich bekannter Weise herstellbar.

EP 0 322 361 A2

**Beschreibung**

**Weitere hydrierte 1-Benzooxacycloalkylpyridincarbonsäureverbindungen**

Die Erfindung betrifft neue hydrierte 1-Benzooxacycloalkylpyridincarbonsäureverbindungen der Formel

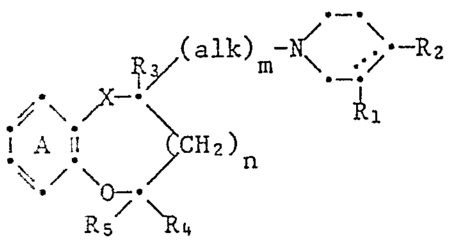

(I),

worin entweder $R_1$ Carboxy, Niederalkoxycarbonyl, amidiertes Carboxy oder gegebenenfalls acyliertes Hydroxymethyl bedeutet und $R_2$ Wasserstoff, eine gegebenenfalls verätherte oder acylierte Hydroxygruppe oder eine gegebenenfalls acylierte Aminogruppe darstellt oder $R_1$ Wasserstoff bedeutet und $R_2$ für Carboxy, Niederalkoxycarbonyl, amidiertes Carboxy oder gegebenenfalls acyliertes Hydroxymethyl steht und worin $R_3$ Wasserstoff oder Niederalkyl bedeutet, $R_4$ Niederalkyl ist, $R_5$ Niederalkyl darstellt, alk Niederalkylen oder Niederalkyliden bedeutet, der Ring A unsubstituiert oder ein- oder mehrfach durch Hydroxy, Niederalkoxy, Niederalkanoyloxy, Cyano, Halogen, Niederalkyl und/oder Trifluormethyl substituiert ist, die gestrichelte Linie zum Ausdruck bringen soll, dass zwischen den Kohlenstoffatomen, welche die Substituenten $R_1$ und $R_2$ tragen, eine Einfach-oder eine Doppelbindung vorliegt; m 0 oder 1 bedeutet und worin entweder X ein Sauerstoffatom oder eine Methylengruppe bedeutet und n für 0 steht oder X für eine direkte Bindung steht und n für 1 steht, und ihre Tautomeren und/oder Salze, die Verwendung dieser Verbindungen, ein Verfahren zu ihrer Herstellung und pharmazeutische Zusammensetzungen, enthaltend eine Verbindung der Formel I oder ein Tautomeres und/oder ein pharmazeutisch verwendbares Salz davon.

Amidiertes Carboxy $R_1$ bzw. $R_2$ weist als Aminogruppe beispielsweise unsubstituiertes oder durch niedere aliphatische Reste mono- oder disubstituiertes Amino auf und bedeutet beispielsweise Carbamyl, N-Niederalkyl- oder N-N-Diniederalkylcarbamyl oder N,N-Niederalkylen-bzw. N,N-(Aza)-, N,N-(Oxa)- oder N,N-(Thia)niederalkylencarbamyl.

Veräthertes Hydroxy $R_2$ ist beispielsweise Niederalkoxy oder gegebenenfalls substituiertes Phenylniederalkoxy.

Acyl in acyliertem Hydroxymethyl $R_1$ bzw. $R_2$ sowie in acyliertem Hydroxy bzw. acyliertem Amino $R_2$ ist beispielsweise von einer organischen Carbon-oder Sulfonsäure abgeleitetes Acyl.

Von einer organischen Carbonsäure abgeleitetes Acyl ist beispielsweise der Rest einer aliphatischen oder monocyclisch-aromatischen Carbonsäure, wie Niederalkanoyl oder gegebenenfalls substituiertes Benzoyl, ferner Pyridoyl.

Von einer organischen Sulfonsäure abgeleitetes Acyl ist beispielsweise Niederalkansulfonyl.

Tautomere Formen von Verbindungen der Formel I existieren z.B. dann, wenn $R_2$ Hydroxy oder Amino bedeutet und die gestrichelte Linie zum Ausdruck bringen soll, dass zwischen den Kohlenstoffatomen, welche die Substituenten $R_1$ und $R_2$ tragen, eine Doppelbindung vorliegt. Die Enole bzw. Enamine der Formel I stehen dann im Gleichgewicht mit den entsprechenden Keto- bzw. Ketimin-Tautomeren der Formel

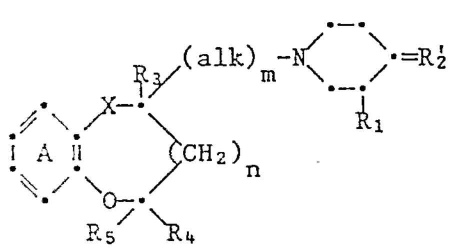

(I'),

worin $R_2'$ Oxo oder Imino bedeutet. Vertreter beider tautomerer Formen können isoliert werden.

Die erfindungsgemässen Verbindungen können ferner in Form von Stereoisomeren vorliegen. Da die Verbindungen der Formel I mindestens ein chirales Kohlenstoffatom (C-Atom) besitzen (z.B das den Rest $R_3$ aufweisende C-Atom), können sie z.B. als reine Enantiomere oder Enantiomerengemische, wie Racemate, und, sofern noch mindestens ein weiteres chirales Zentrum vorhanden ist (z.B. das Atom $C_4$ eines durch von

Wasserstoff verschiedenes $R_2$ 4-substituierten Piperidinrestes und/oder das Atom $C_3$ eines durch von Wasserstoff verschiedenes $R_1$ 3-substituierten Piperidinrestes), auch als Diastereomere, Diastereomerengemische oder Racematgemische vorliegen. So können beispielsweise im Hinblick auf $R_1$ und $R_2$ geometrische Isomere, wie cis- und trans-Isomere, gebildet werden, falls $R_1$ und $R_2$ von Wasserstoff verschieden sind und die gestrichelte Linie bedeutet, dass zwischen den Kohlenstoffatomen, welche die Substituenten $R_1$ und $R_2$ tragen, eine Einfachbindung vorliegt.

Salze von Verbindungen der Formel I bzw. deren Tautomeren sind insbesondere entsprechende Säureadditionssalze, vorzugsweise pharmazeutisch verwendbare Säureadditionssalze. Diese werden beispielsweise mit starken anorganischen Säuren, wie Mineralsäuren, z.B. Schwefelsäure, einer Phosphorsäure oder einer Halogenwasserstoffsäure, mit starken organischen Carbonsäuren, wie Niederalkancarbonsäuren, z.B. Essigsäure, wie gegebenenfalls ungesättigten Dicarbonsäuren, z.B. Malon-, Malein-oder Fumarsäure, oder wie Hydroxycarbonsäuren, z.B. Wein- oder Zitronensäure, oder mit Sulfonsäuren, wie Niederalkan- oder gegebenenfalls substituierten Benzolsulfonsäuren, z.B. Methan- oder p-Toluolsulfonsäure, gebildet. Bedeutet $R_1$ oder $R_2$ beispielsweise Carboxy, können entsprechende Verbindungen Salze mit Basen bilden. Geeignete Salze mit Basen sind beispielsweise entsprechende Alkalimetall- oder Erdalkalimetallsalze, z.B. Natrium-, Kalium- oder Magnesiumsalze, pharmazeutisch verwendbare Uebergangsmetallsalze, wie Zink- oder Kupfersalze, oder Salze mit Ammoniak oder organischen Aminen, wie cyclische Amine, wie Mono-, Di-bzw. Triniederalkylamine, wie Hydroxyniederalkylamine, z.B. Mono-, Di-bzw. Trihydroxyniederalkylamine, Hydroxyniederalkyl-niederalkyl-amine oder Polyhydroxyniederalkylamine. Cyclische Amine sind z.B. Morpholin, Thiomorpholin, Piperidin oder Pyrrolidin. Als Mononiederalkylamine kommen beispielsweise Aethyl- oder t-Butylamin, als Diniederalkylamin beispielsweise Diäthyl- oder Diisopropylamin, als Triniederalkylamine beispiels weise Trimethyl- oder Triäthylamin in Betracht. Entsprechende Hydroxyniederalkylamine sind z.B. Mono-, Di- bzw. Triäthanolamin, und Hydroxyniederalkyl-niederalkyl-amine sind z.B. N,N-Dimethylamino- oder N,N-Diäthylamino-äthanol, als Polyhydroxyniederalkylamin kommt z.B. Glucosamin in Frage.

Umfasst sind ferner für pharmazeutische Verwendungen ungeeignete Salze, da diese beispielsweise für die Isolierung bzw. Reinigung freier erfindungsgemässer Verbindungen sowie deren pharmazeutisch verwendbarer Salze verwendet werden können.

Vor- und nachstehend sind unter mit "Nieder" bezeichneten Resten oder Verbindungen, sofern nicht abweichend definiert, insbesondere solche zu verstehen, die bis und mit 7, vor allem bis und mit 4, Kohlenstoffatome enthalten.

Niederalkoxy ist z.B. $C_1$-$C_4$-Alkoxy, wie Methoxy, Aethoxy, n-Propyloxy, Isopropyloxy, n-Butyloxy, Isobutyloxy oder tert-Butyloxy.

Niederalkyl ist z.B. $C_1$-$C_4$-Alkyl, wie Methyl, Aethyl, n-Propyl, Isopropyl, n-Butyl, Isobutyl, sek-Butyl oder tert-Butyl, und umfasst ferner $C_5$-$C_7$-Alkyl-, d.h. Pentyl-, Hexyl- oder Heptylreste.

Niederalkylen alk ist z.B. $C_1$-$C_4$-Alkylen, welches die beiden in Formel I eingezeichneten Ringsysteme in erster Linie durch bis und mit 3 C-Atome überbrückt, und kann z.B. Methylen, Aethylen oder 1,3-Propylen, aber auch 1,2-Propylen, 1,2- oder 1,3-(2-Methyl)-propylen oder 1,2- oder 1,3-Butylen sein, kann aber ferner die beiden Ringsysteme auch durch 4 C-Atome überbrücken, d.h. 1,4-Butylen darstellen.

Niederalkyliden alk ist z.B. $C_1$-$C_4$-Alkyliden und kann z.B. Methylen, Aethyliden, 1,1- oder 2,2-Propyliden oder 1,1- oder 2,2-Butyliden sein.

Niederalkanoyl ist z.B. $C_2$-$C_5$-Alkanoyl, wie Acetyl, Propionyl, Butyryl, Isobutyryl oder Pivaloyl.

Niederalkanoyloxy ist z.B. $C_2$-$C_5$-Alkanoyloxy, wie Acetyloxy, Propionyloxy, Butyryloxy, Isobutyryloxy oder Pivaloyloxy.

Niederalkoxycarbonyl ist z.B. $C_2$-$C_5$-Alkoxycarbonyl, wie Methoxy-, Aethoxy-, n-Propyloxy-, Isopropyloxy-, n-Butyloxy-, Isobutyloxy-oder tert.-Butyloxycarbonyl.

N-Niederalkylcarbamyl ist z.B. N-$C_1$-$C_4$-Alkylcarbamyl, wie N-Methyl-, N-Aethyl-, N-(n-Propyl)-, N-Isopropyl-, N-(n-Butyl)-, N-Isobutyl-oder N-tert.-Butylcarbamyl.

N,N-Diniederalkylcarbamyl ist z.B. N,N-Di-$C_1$-$C_4$-alkylcarbamyl, wobei jeweils die beiden N-Alkylgruppen gleich oder verschieden sein können, wie N,N-Dimethyl-, N,N-Diäthyl-, N,N-Diisopropyl- oder N-Butyl-N-methylcarbamyl.

N,N-Niederalkylen- bzw. N,N-(Aza)-, N,N-(Oxa)- oder N,N-(Thia)-niederalkylencarbamyl weist beispielsweise 3 bis und mit 8, insbesondere 5 oder 6, Ringglieder auf und bedeutet beispielsweise Pyrrolidino-, Piperidino-, Piperazino- bzw. N′-Niederalkyl-, wie N′-Methylpiperazino-, Morpholino-oder Thiomorpholinocarbonyl.

Gegebenenfalls substituiertes Phenylniederalkoxy ist z.B. gegebenenfalls im Phenylteil substituiertes Phenyl-$C_1$-$C_4$-alkoxy, wie Benzyloxy, p-Chlorbenzyloxy, 1-Phenyläthoxy oder 1-(p-Bromphenyl)-n-butyloxy.

Gegebenenfalls subbstituiertes Benzoyl ist z.B. Benzoyl, p-Chlorbenzoyl oder p-Nitrobenzoyl.

Niederalkansulfonyl ist z.B. $C_1$-$C_4$-Alkansulfonyl, wie Methan- oder Aethansulfonyl.

Halogen ist insbesondere Halogen mit einer Atomnummer bis und mit 35, wie Fluor, Chlor oder Brom, und umfasst ferner Iod.

Die verbindungen der Formel I, ihre Tautomeren und/oder ihre pharmazeutisch verwendbaren Salze weisen beispielsweise wertvolle pharmakologische, insbesondere nootrope, Eigenschaften auf. So bewirken sie z.B. im Two-Compartment Passive Avoidance-Testmodell nach Mondadori und Classen, Acta Neurol. Scand. 69, Suppl. 99, 125 (1984) in Dosismengen ab etwa 0,1 mg/kg i.p. sowie p.o. bei der Maus eine Reduktion der amnesischen Wirkung eines zerebralen Elektroschocks.

Ebenso besitzen die erfindungsgemässen Verbindungen eine beträchtliche gedächtnisverbessernde Wirkung, die im Step-down Passive Avoidance-Test nach Mondadori und Waser, Psychopharmacol. 63, 297 (1979) ab einer Dosis von etwa 0,1 mg/kg i.p. sowie p.o. an der Maus festzustellen ist.

Entsprechend können die Verbindungen der Formel I bzw. deren Tautomere und/oder ihre pharmazeutisch verwendbaren Salze als Pharmazeutika, z.B. Nootropika, beispielsweise zur therapeutischen und/oder prophylaktischen Behandlung von cerebralen Insuffizienzerscheinungen, insbesondere Gedächtnisstörungen, verwendet werden. Ein weiterer Gegenstand der Erfindung ist somit die Verwendung von Verbindungen der Formel I, ihrer Tautomeren und/oder ihrer pharmazeutisch verwendbaren Salze zur Herstellung von Arzneimitteln, insbesondere von Nootropika, zur Behandlung von cerebralen Insuffizienzerscheinungen, insbesondere Gedächtnisstörungen. Dabei kann auch die gewerbsmässige Herrichtung der Wirksubstanzen eingeschlossen sein.

Die Erfindung betrifft in erster Linie Verbindungen der Formel I, worin entweder $R_1$ Carboxy, Niederalkoxycarbonyl, Carbamyl, N-Niederalkylcarbamyl, N,N-Diniederalkylcarbamyl, N,N-Niederalkylencarbamyl, N,N-(Aza)-, N,N-(Oxa)- oder N,N-(Thia)niederalkylencarbamyl, Hydroxymethyl, Niederalkanoyloxymethyl, Niederalkansulfonyloxymethyl, Benzoyloxymethyl oder Pyridoyloxymethyl bedeutet und $R_2$ Wasserstoff, Hydroxy, Niederalkoxy, Benzyloxy, Niederalkanoyloxy, Niederalkansulfonyloxy, Benzoyloxy, Pyridoyloxy, Amino, Niederalkanoylamino, Niederalkansulfonylamino, Benzoylamino oder Pyridoylamino darstellt oder $R_1$ Wasserstoff bedeutet und $R_2$ für Carboxy, Niederalkoxycarbonyl, Carbamyl, N-Niederalkylcarbamyl, N,N-Diniederalkylcarbamyl, N,N-Niederalkylencarbamyl, N,N-(Aza)-, N,N-(Oxa)- oder N,N-(Thia)niederalkylencarbamyl, Hydroxy methyl, Niederalkanoyloxymethyl, Niederalkansulfonyloxymethyl, Benzoyloxymethyl oder Pyridoyloxymethyl steht und worin $R_3$ Wasserstoff oder Niederalkyl bedeutet, $R_4$ Niederalkyl ist, $R_5$ Niederalkyl darstellt, alk Niederalkylen, das die beiden in Formel I eingezeichneten Ringsysteme durch bis und mit 3 C-Atome überbrückt, oder Niederalkyliden darstellt, der Ring A unsubstituiert ist oder ein-, zwei- oder mehrfach durch Hydroxy, Niederalkoxy, Niederalkanoyloxy, Cyano, Halogen, Niederalkyl und/oder Trifluormethyl substituiert ist, die gestrichelte Linie zum Ausdruck bringen soll, dass zwischen den Kohlenstoffatomen, welche die Substituenten $R_1$ und $R_2$ tragen, eine Einfach- oder eine Doppelbindung vorliegt, m 0 oder 1 bedeutet und worin entweder X ein Sauerstoffatom oder eine Methylengruppe bedeutet und n für 0 steht oder X für eine direkte Bindung steht und n für 1 steht, und ihre Tautomeren und/oder Salze.

Die Erfindung betrifft vor allem Verbindungen der Formel I, worin $R_1$ $C_1$-$C_4$-Alkoxycarbonyl, wie Methoxy- oder Aethoxycarbonyl, oder Carbamyl bedeutet, $R_2$ Wasserstoff oder Hydroxy bedeutet, $R_3$ Wasserstoff ist, $R_4$ $C_1$-$C_4$-Alkyl, wie Methyl, bedeutet, $R_5$ $C_1$-$C_4$-Alkyl, wie Methyl, darstellt, alk für $C_1$-$C_4$-Alkylen, welches die beiden in Formel I eingezeichneten Ringsysteme durch bis und mit 3 C-Atome überbrückt, wie Methylen oder Aethylen, steht, der Ring A unsubstituiert oder, insbesondere in 6-Stellung, durch $C_1$-$C_4$-Alkoxy, wie Methoxy, $C_1$-$C_4$-Alkyl, wie Methyl, Halogen mit einer Atomnummer bis und mit 35, wie Fluor oder Chlor, Cyano oder Trifluormethyl substituiert ist, die gestrichelte Linie zum Ausdruck bringen soll, dass zwischen den Kohlenstoffatomen, welche die Substituenten $R_1$ und $R_2$ tragen, eine Einfach- oder eine Doppelbindung vorliegt, m für 1 steht, X ein Sauerstoffatom oder eine Methylengruppe bedeutet und n für 0 steht, und ihre Tautomeren und/oder Salze.

Die Erfindung betrifft insbesondere verbindungen der Formel I, worin $R_1$ $C_1$-$C_4$-Alkoxycarbonyl, wie Methoxycarbonyl, bedeutet, $R_2$ Wasserstoff darstellt, $R_3$ Wasserstoff ist, $R_4$ $C_1$-$C_4$-Alkyl, wie Methyl, bedeutet, $R_5$ $C_1$-$C_4$-Alkyl, wie Methyl, darstellt, alk für Aethylen steht, der Ring A unsubstituiert oder, insbesondere in 6-Stellung, einfach durch Halogen mit einer Atomnummer bis und mit 35, wie Fluor oder Chlor, substituiert ist, die gestrichelte Linie zum Ausdruck bringen soll, dass zwischen den Kohlenstoffatomen, welche die Substituenten $R_1$ und $R_2$ tragen, eine Doppelbindung vorliegt, m für 1 steht, X eine Methylengruppe darstellt und n für 0 steht, und ihre Salze.

Die Erfindung betrifft in allererster Linie Verbindungen der Formel I, worin $R_1$ $C_1$-$C_4$-Alkoxycarbonyl, wie Methoxycarbonyl, bedeutet, $R_2$ Wasserstoff darstellt, $R_3$ Wasserstoff ist, $R_4$ $C_1$-$C_4$-Alkyl, wie Methyl, bedeutet, $R_5$ $C_1$-$C_4$-Alkyl, wie Methyl, darstellt, alk für Aethylen steht, der Ring A unsubstituiert ist, die gestrichelte Linie zum Ausdruck bringen soll, dass zwischen den Kohlenstoffatomen, welche die Substituenten $R_1$ und $R_2$ tragen, eine Doppelbindung vorliegt, m für 1 steht, X eine Methylengruppe darstellt und n für 0 steht, und ihre Salze.

Die Erfindung betrifft namentlich die in den Beispielen genannten neuen Verbindungen der Formel I und ihre Salze und Verfahren zu ihrer Herstellung.

Ebenfalls Gegenstand der vorliegenden Erfindung ist ein Verfahren zur Herstellung von Verbindungen der Formel I oder ihrer Tautomeren und/oder Salze, z.B. dadurch gekennzeichnet, dass man

a) eine Verbindung der Formel

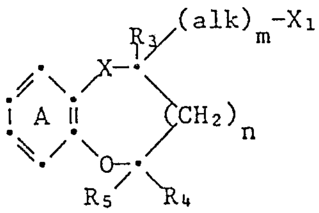

(IIa)

oder ein Salz davon, worin $X_1$ Hydroxy oder reaktionsfähiges verestertes Hydroxy bedeutet, mit einer Verbindung der Formel

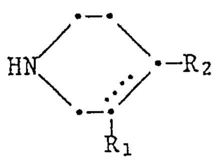

(IIb)

oder einem Tautomeren und/oder Salz davon umsetzt oder

b) in einer Verbindung der Formel

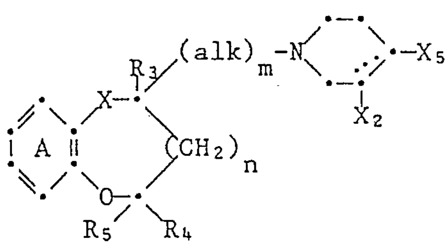

(III)

oder einem Tautomeren und/oder Salz davon, worin $X_2$ einen in von Wasserstoff verschiedenes $R_1$ überführbaren Rest bedeutet und $X_5$ einen Rest $R_a$ darstellt, wobei $R_a$ Wasserstoff, eine gegebenenfalls verätherte oder acylierte Hydroxygruppe oder eine gegebenenfalls acylierte Aminogruppe ist, $X_2$ in von Wasserstoff verschiedenes $R_1$ überführt oder in einer Verbindung der Formel III oder einem Tautomeren und/oder Salz davon, worin $X_2$ Wasserstoff ist und $X_5$ einen in $R_b$ überführbaren Rest bedeutet, wobei $R_b$ für einen von einem Rest $R_a$ verschiedenen Rest $R_2$ steht, $X_5$ in $R_b$ überführt oder

c) zur Herstellung einer Verbindung der Formel I oder eines Tautomeren und/oder Salzes davon, worin $R_2$ Hydroxy oder Amino bedeutet und worin $R_1$ von Wasserstoff und gegebenenfalls acyliertem Hydroxymethyl verschieden ist, eine Verbindung der Formel

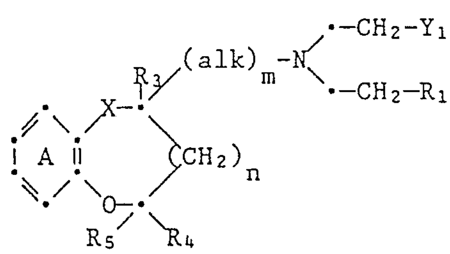

(IV),

worin $Y_1$ eine Gruppe der Formel $-CH=R_2'$, $-C(Y_2)=R_2'$, $-CH(Y_2)-R_2$ oder Cyano darstellt, wobei $R_2'$ Oxo oder Imino bedeutet und $Y_2$ einen abspaltbaren Rest darstellt, oder ein Salz davon cyclisiert oder

d) zur Herstellung einer Verbindung der Formel I′, worin $R_2'$ Oxo oder Imino bedeutet und $R_1$ von Wasserstoff verschieden ist, oder eines Tautomeren und/oder Salzes davon eine Verbindung der Formel

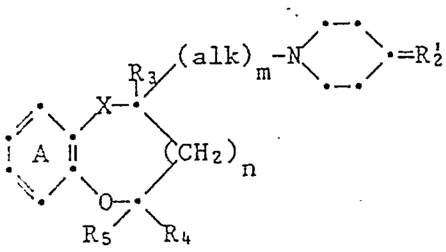

(Va)

oder ein Tautomeres und/oder ein Salz davon mit einer Verbindung der Formel

$X_3$-$R_1$ (Vb)

oder einem Salz davon, worin $R_1$ von Wasserstoff verschieden ist und $X_3$ Halogen oder Niederalkoxy bedeutet, umsetzt oder

e) zur Herstellung einer Verbindung der Formel I oder eines Tautomeren und/oder Salzes davon, worin $R_2$ eine gegebenenfalls verätherte oder acylierte Hydroxygruppe oder eine gegebenenfalls acylierte Aminogruppe ist, in einer Verbindung der Formel

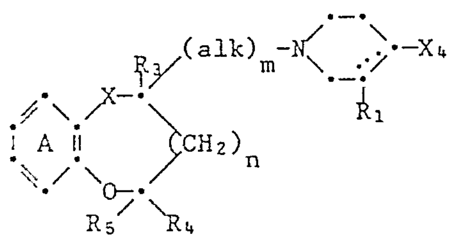

(VI)

oder einem Salz davon, worin $X_4$ einen in $R_2$ überführbaren Rest bedeutet, $X_4$ in $R_2$ überführt oder

f) in einem Salz der Formel

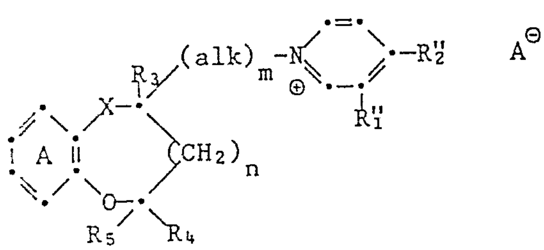

(VII),

worin $A^{\ominus}$ für das Anion einer Säure steht, $R_1''$ ein Rest $R_1$ oder veräthertes oder geschütztes Hydroxymethyl ist und $R_2''$ einen Rest $R_2$, geschütztes Hydroxy, geschütztes Amino oder veräthertes oder geschütztes Hydroxymethyl bedeutet, die überschüssigen Doppelbindungen zu Einfachbindungen reduziert und, falls $R_1''$ von $R_1$ und/oder $R_2''$ von $R_2$ verschieden ist, $R_1''$ in $R_1$ und/oder $R_2''$ in $R_2$ überführt oder

g) zur Herstellung einer Verbindung der Formel I oder eines Tautomeren und/oder Salzes davon, worin $R_2$ Carboxy, amidiertes Carboxy oder Niederalkoxycarbonyl bedeutet und die gestrichelte Linie zum Ausdruck bringen soll, dass zwischen den Kohlenstoffatomen, welche die Substituenten $R_1$ und $R_2$ tragen, eine Einfachbindung vorliegt, eine Verbindung der Formel

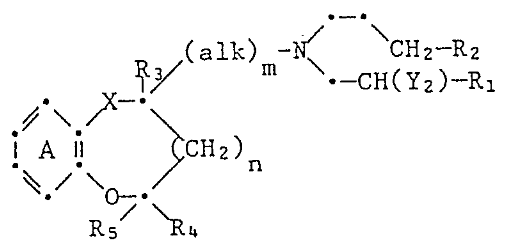

(VIII),

worin $Y_2$ einen abspaltbaren Rest darstellt, oder ein Salz davon cyclisiert und jeweils eine gegebenenfalls vorhandene Schutzgruppe abspaltet und jeweils gewünschtenfalls eine verfahrensgemäss oder auf andere Weise erhältliche Verbindung der Formel I in eine andere Verbindung der Formel I überführt, jeweils ein verfahrensgemäss erhältliches Isomerengemisch in die Komponenten auftrennt, jeweils ein verfahrensgemäss erhältliches Enantiomeren- bzw. Diastereomerengemisch in die Enantiomeren bzw. Diastereomeren aufspaltet und/oder jeweils eine verfahrensgemäss erhältliche freie Verbindung der Formel I in ein Salz überführt oder ein verfahrensgemäss erhältliches Salz in die freie Verbindung der Formel I oder in ein anderes Salz umwandelt.

Die vor- und nachstehend in den Varianten beschriebenen Umsetzungen werden in an sich bekannter Weise durchgeführt, z.B. in Ab- oder üblicherweise in Anwesenheit eines geeigneten Lösungs- oder Verdünnungsmittels oder eines Gemisches derselben, wobei man je nach Bedarf unter Kühlen, bei Raumtemperatur oder unter Erwärmen z.B. in einem Temperaturbereich von etwa -10° bis zur Siedetemperatur des Reaktionsmediums, vorzugsweise von etwa 20° bis etwa 150°C, und, falls erforderlich, in einem geschlossenen Gefäss, unter Druck, in einer Inertgasatmosphäre und/oder unter wasserfreien Bedingungen arbeitet.

Das vor- und nachstehend aufgeführte Ausgangsmaterial der Formeln IIa und IIb, III, IV, Va und Vb, VI, VII und VIII, das für die Herstellung der Verbindungen der Formel I, deren Tautomeren und Salzen entwickelt wurde, ist zum Teil bekannt oder kann ebenfalls nach an sich bekannten Methoden, z.B. analog den vorstehend beschriebenen Verfahrensvarianten, hergestellt werden.

Ausgangsmaterial mit basischen Zentren kann z.B. in Form von Säureadditionssalzen, beispielsweise mit den vorstehend aufgeführten Säuren, vorliegen, während Ausgangsverbindungen mit sauren Gruppen, Salze mit Basen, z.B. der vorstehend genannten Art, bilden können. Weiter können Ausgangsverbindungen in Form von Tautomeren vorliegen, insbesondere Verbindungen der Formel IIb, wenn $R_2$ Hydroxy bedeutet und die gestrichelte Linie zum Ausdruck bringen soll, dass zwischen den Kohlenstoffatomen, welche die Substituenten $R_1$ und $R_2$ tragen, eine Doppelbindung vorliegt.

Variante a):

Reaktionsfähiges verestertes Hydroxy bedeutet insbesondere mit einer starken anorganischen Säure oder organischen Sulfonsäure verestertes Hydroxy, beispielsweise Halogen, wie Chlor, Brom oder Iod, Sulfonyloxy, wie Hydroxysulfonyloxy, Halogensulfonyloxy, z.B. Fluorsulfonyloxy, gegebenenfalls, z.B. durch Halogen, substituiertes Niederalkansulfonyloxy, z.B. Methan- oder Trifluormethansulfonyloxy, Cycloalkansulfonyloxy, z.B. Cyclohexansulfonyloxy, oder gegebenenfalls, z.B. durch Niederalkyl oder Halogen, substituiertes Benzolsulfonyloxy, z.B. p-Bromphenyl- oder p-Toluolsulfonyloxy.

Die N-Alkylierung wird insbesondere in Gegenwart eines Kondensationsmittels, wie einer geeigneten Base, durchgeführt. Als Basen kommen beispielsweise Alkalimetallhydroxide, -hydride, -amide, -alkanolate, -carbonate, -triphenylmethylide, -diniederalkylamide, -aminoniederalkylamide oder -niederalkylsilylamide, Naphthalinamine, Niederalkylamine, basische Heterocyclen, Ammoniumhydroxide sowie carbocyclische Amine in Frage. Beispielhaft seien Natriumhydroxid, -hydrid, -amid, -äthylat, Kalium-tert-butylat, -carbonat, Lithiumtriphenylmethylid, Lithium-diisopropylamid, Kalium-3-(aminopropyl)-amid, -bis-(trimethylsilyl)-amid, Dimethylaminonaphthalin, Di- oder Triäthylamin, Pyridin, Benzyl-trimethyl-ammoniumhydroxid, 1,5-Diaza-bicyclo[4.3.0]non-5-en (DBN) sowie 1,5-Diaza-bicyclo[5.4.0]undec-5-en (DBU) genannt.

Die Ausgangsmaterialien der Formeln IIa und IIb sind teilweise bekannt oder lassen sich in Analogie zu den bekannten Ausgangsmaterialien herstellen.

Variante b):

Ein in von Wasserstoff verschiedenes $R_1$ überführbarer Rest $X_2$ bzw. ein in einen Rest $R_b$ überführbarer Rest $X_5$ ist beispielsweise von $R_1$ bzw. $R_b$ verschiedenes funktionell abgewandeltes Carboxy, wie Cyano, anhydridisiertes Carboxy, gegebenenfalls substituiertes Amidino, gegebenenfalls verestertes oder anhydridisiertes Carboximidoyl, von verestertem oder amidiertem Carboxy $R_1$ bzw. $R_2$ verschiedenes verestertes oder amidiertes Carboxy, Triniederalkoxy- oder Trihalogenmethyl.

Anhydridisiertes Carboxy ist beispielsweise mit einer Mineralsäure, wie Halogenwasserstoffsäure, oder mit einer Carbonsäure, wie einer gegebenenfalls substituierten Niederalkan- bzw. Benzoesäure oder einem Kohlensäurehalogenid-niederalkylhalbester anhydridisiertes Carboxy. Als Beispiele seien Halogencarbonyl, wie Chlorcarbonyl, Niederalkanoyloxycarbonyl, wie Acetyloxycarbonyl, oder Niederalkoxycarbonyloxycarbonyl, wie Aethoxycarbonyloxycarbonyl, genannt.

Substituiertes Amidino ist beispielsweise mit einem aliphatischen Rest, z.B. Niederalkyl, substituiertes Amidino, wie Niederalkylamidino, z.B. Aethylamidino.

Unter verestertem oder anhydridisiertem Carboximidoyl ist z.B. Alkoxy-, bzw. Halogencarboximidoyl, beispielsweise Niederalkoxy-, wie Aethoxy-, bzw. Chlor-carboximidoyl, zu verstehen.

Triniederalkoxy- bzw. Trihalogenmethyl ist z.B. Trimethoxymethyl bzw. Trichlormethyl.

Reste $R_b$ sind beispielsweise Carboxy-, Niederalkoxycarbonyl-, amidierte Carboxy- oder gegebenenfalls acylierte Hydroxymethylreste $R_2$.

$X_2$ kann beispielsweise durch Solvolyse in von Wasserstoff verschiedenes R überführt werden, ebenso wie $X_5$ beispielsweise durch Solvolyse in einen Rest $R_b$ überführbar ist. Solvolysemittel sind beispielsweise Wasser, dem gewünschten veresterten Carboxy $R_1$ bzw. $R_2$ entsprechende Niederalkanole, Ammoniak oder der gewünschten amidierten Carboxygruppe $R_1$ bzw. $R_2$ entsprechende Amine. Die Behandlung mit einem entsprechenden Solvolysemittel wird gegebenenfalls in Gegenwart einer Säure oder Base durchgeführt. Als Säuren kommen beispielsweise anorganische oder organische Protonsäuren, wie Mineralsäuren z.B. Schwefelsäure oder Halogenwasserstoffsäuren, beispielsweise Chlorwasserstoffsäure, wie Sulfonsäuren, z.B. Niederalkan- oder gegebenenfalls substituierte Benzolsulfonsäure, beispielsweise Methan- oder p-Toluolsulfonsäure, oder wie Carbonsäuren, z.B. Niederalkancarbonsäuren, beispielsweise Essigsäure, in Frage, während als Basen beispielsweise die unter Variante a) genannten verwendet werden können, insbesondere Natrium- oder Kaliumhydroxid.

Bei der Solvolyse wird die Cyanogruppe, anhydridisiertes Carboxy, gegebenenfalls substituiertes Amidino, gegebenenfalls verestertes oder anhydridisiertes Carboximidoyl, von verestertem oder amidiertem Carboxy $R_1$ bzw. $R_2$ verschiedenes verestertes oder amidiertes Carboxy, Triniederalkoxy- oder Trihalogenmethyl zu Carboxy hydrolysiert. Dabei können gegebenenfalls am Ring A befindliche Niederalkanoyloxyreste im Verlauf der Hydrolyse zu Hydroxy hydrolysiert werden.

Cyano, anhydridisiertes Carboxy, von verestertem oder amidiertem Carboxy $R_1$ bzw. $R_2$ verschiedenes verestertes oder amidiertes Carboxy werden beispielsweise mit einem geeigneten Niederalkanol zu verestertem Carboxy $R_1$ bzw. $R_2$ alkoholysiert und Cyano und anhydridisiertes Carboxy beispielsweise mit Ammoniak oder einem dem amidierten Carboxy $R_1$ bzw. $R_2$ entsprechenden Amin ammono- bzw. aminolysiert.

Das Ausgangsmaterial der Formel III kann beispielsweise in Analogie zu der unter Variante a) beschriebenen Weise durch Umsetzung einer Verbindung der Formel

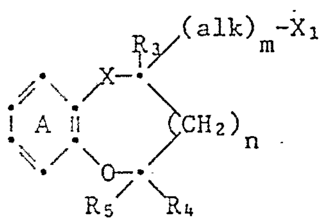

(IIa)

mit einer Verbindung der Formel

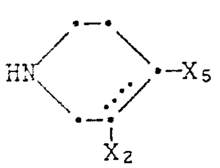

(IIIa)

oder einem Tautomeren und/oder Salz davon in Gegenwart einer der genannten Basen hergestellt werden.

Verbindungen der Formel III, worin $X_2$ einen in von Wasserstoff verschiedenes $R_1$ überführbaren Rest bedeutet und $X_5$ Hydroxy oder Amino darstellt, können vorteilhaft auch durch Cyclisierung einer Verbindung der Formel

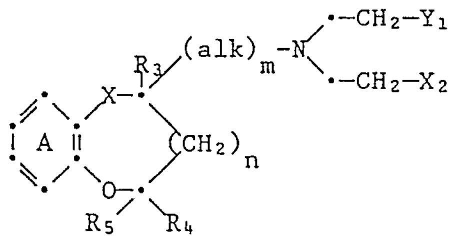

(IVg),

worin $Y_1$ eine Gruppe der Formel $-CH{=}R_2'$, $-C(Y_2){=}R_2'$, $-CH(Y_2)-R_2$ oder Cyano darstellt, wobei $R_2'$ Oxo oder Imino bedeutet, $R_2$ Hydroxy oder Amino ist und $Y_2$ einen abspaltbaren Rest darstellt, oder eines Salzes davon hergestellt werden, wobei z.B. in analoger Weise wie unter der Verfahrensvariante c) angegeben gearbeitet wird.

Variante c):

Abspaltbare Reste $Y_2$ in Gruppen der Formel $-C(Y_2){=}R_2'$ oder $-CH(Y_2)-R_2$ sind beispielsweise reaktionsfähige veresterte Hydroxygruppen, wie mit einer starken anorganischen Säure oder organischen Sulfonsäure verestertes Hydroxy, beispielsweise Halogen, wie Chlor, Brom oder Iod, Sulfonyloxy, wie Hydroxysulfonyloxy, Halogensulfonyloxy, z.B. Fluorsulfonyloxy, gegebenenfalls, z.B. durch Halogen, substituiertes Nieder alkansulfonyloxy z.B. Methan- oder Trifluormethansulfonyloxy, Cycloalkansulfonyloxy, z.B. Cyclohexansulfonyloxy, oder gegebenenfalls, z.B. durch Niederalkyl oder Halogen, substituiertes Benzolsulfonyloxy, z.B. p-Bromphenyl- oder p-Toluolsulfonyloxy, oder verätherte Hydroxygruppen, beispielsweise Niederalkoxy oder gegebenenfalls substituiertes Phenylniederalkoxy.

Die Cyclisierung kann beispielsweise in Analogie zur Dieckmann-Reaktion, insbesondere in Gegenwart einer der unter der Variante a) genannten Basen, und unter anschliessender hydrolytischer Aufarbeitung, durchgeführt werden.

In einer bevorzugten Ausführungsform kann man beispielsweise eine Verbindung der Formel

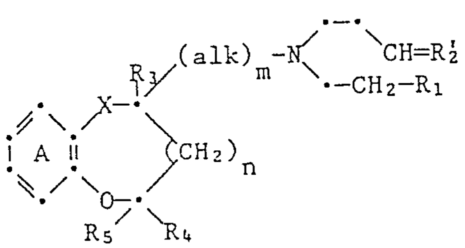

(IVa),

worin $R_2'$ Oxo oder Imino bedeutet, der Behandlung mit einer der genannten Basen, insbesondere mit einem Alkalimetallniederalkanolat, z.B. mit Natriummethanolat oder Natriumäthanolat, unterwerfen. Dabei cyclisiert die Verbindung IVa zu einer Verbindung der Formel I, worin die gestrichelte Linie anzeigt, dass zwischen den Kohlenstoffatomen, welche die Substituenten $R_1$ und $R_2$ tragen, eine Einfachbindung vorliegt und $R_2$ Hydroxy oder Amino bedeutet. Ausgangsstoffe IVa werden z.B. erhalten, indem man einen reaktionsfähigen Alkylester der Formel

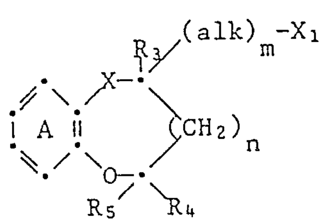

(IIa),

worin $X_1$ reaktionsfähiges verestertes Hydroxy ist, mit einer Verbindung der Formel $H_2N-CH_2-CH_2-R_1$ (IVb) und das erhaltene Zwischenprodukt der Formel

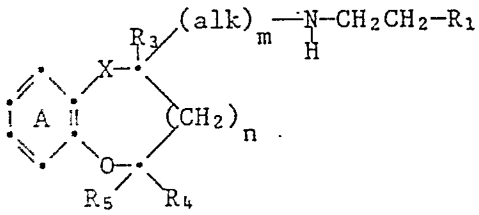

(IVc)

mit Acrolein oder einem gegebenenfalls funktionell abgewandelten Aldehyd der Formel $Y_1$-$CH_2$-$CH_2$-$CH=R_2'$ (IVd; $Y_1$ = reaktionsfähiges verestertes Hydroxy; $R_2'$ = Oxo oder Imino) umsetzt.

In einer anderen bevorzugten Ausführungsform der Variante c) cyclisiert man eine Verbindung der Formel IV, worin $Y_1$ und $R_1$ Niederalkoxycarbonyl bedeuten, d.h. worin $Y_1$ eine Gruppe der Formel -$C(Y_2)=R_2'$ bedeutet, in welcher $R_2'$ Oxo ist und der abspaltbare Rest $Y_2$ als veräthertes Hydroxy eine Niederalkoxygruppe aufweist, zu der entsprechenden Verbindung der Formel I', worin $R_2'$ Oxo ist und $R_1$ Niederalkoxycarbonyl bedeutet.

Zur Herstellung der letztgenannten Ausgangsverbindungen der Formel IV kann man beispielsweise von Verbindungen der Formel

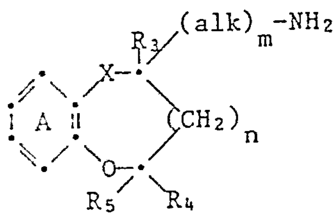

(IVe)

oder deren Salzen ausgehen, welche z.B. durch Reduktion der entsprechen den Nitrile erhältlich sind, und diese mit mindestens 2 Mol einer Verbindung der Formel

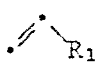

(IVf)

zur Reaktion bringen.

Variante d):

Die verfahrensgemässe C-Acylierung kann insbesondere in Gegenwart einer der unter der Variante a) genannten Basen, besonders vorteilhaft jedoch mittels einer Metallbase, wie Lithiumdiisopropylamin oder n-Butyllithium, gegebenenfalls in Gegenwart von Chlortrimethylsilan, erfolgen.

Die Umsetzung einer Verbindung der Formel

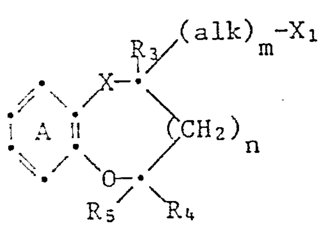

(IIa)

mit einer Verbindung der Formel

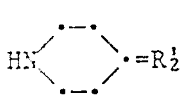

(Vc)

oder einem Salz davon führt in Analogie zu der N-Alkylierung gemäss Variante a) in Gegenwart einer der

erwähnten Basen zu dem Ausgangsmaterial der Formel Va.

Variante e):

In einen Rest $R_2$ der angegebenen Art überführbare Reste $X_4$ sind beispielsweise durch Solvolyse, d.h. Umsetzung mit einer entsprechenden Verbindung der Formel $R_2H$ oder einem Salz davon, in eine derartige Gruppe $R_2$ überführbare Reste, beispielsweise Halogenatome, z.B. Chlor, Brom oder Iod. In Hydroxy $R_2$ überführbare Reste $X_4$ sind ferner Diazoniumgruppen, z.B. der Formel $-N_2^{\oplus}A^{\ominus}$, worin $A^{\ominus}$ das Anion einer starken Säure, wie einer Mineralsäure, z.B. das Chlorid- oder Sulfation, bedeutet.

Die Solvolyse erfolgt in üblicher Weise, beispielsweise in Gegenwart einer Base, wie eines Alkalimetall- oder Erdalkalimetallhydroxides, z.B. von Natrium- oder Kaliumhydroxid, oder einer tertiären Stickstoffbase, z.B. eines Triniederalkylamins, wie Triäthylamin, oder einer heteroaromatischen Stickstoffbase, wie Pyridin, bzw. eines quaternären Ammoniumhydroxides, wie Benzyl-trimethyl-ammoniumhydroxid, oder indem man die Verbindung $R_2H$ in Form eines Metallsalzes, z.B. der Formel $R_2^{\ominus}M^{\oplus}$, worin $M^{\oplus}$ ein Alkalimetallkation, wie das Natriumion, bedeutet, einsetzt. Vorteilhaft arbeitet man in Gegenwart eines Lösungs- oder verdünnungsmittels, z.B. in einem Ueberschuss der Reaktionskomponente $R_2H$ und/oder einem mit dieser mischbaren inerten Lösungsmittel, erforderlichenfalls unter Kühlen oder Erwärmen, z.B. im Temperaturbereich von etwa 0° bis 120°C, und/oder unter Inertgas, wie Stickstoff.

Die Solvolyse von Resten $X_4$ zu derartigen Gruppen $R_2$ kann gegebenenfalls mit der solvolytischen Ueberführung von solvolysierbaren Gruppen $R_1$ in andere erfindungsgemässe Gruppen $R_1$ kombiniert werden, beispielsweise können bei der Ammonolyse von Resten $X_4$ zu Amino $R_2$ gegebenenfalls auch Niederalkoxycarbonylgruppen $R_1$ oder andere zu Carbamyl $R_1$ solvolysierbare Gruppen $R_1$ gleichzeitig zu Carbamylgruppen $R_1$ ammonolysiert werden.

Zur Herstellung von Ausgangsverbindungen der Formel VI und deren Salzen geht man beispielsweise von Verbindungen der Formel IIa

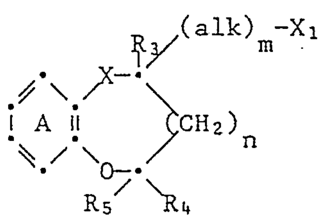

(IIa)

aus und setzt diese mit einer entsprechenden Verbindung der Formel

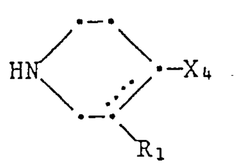

(VIa)

oder einem Salz davon in Gegenwart einer der vorstehend genannten Basen um, wobei z.B. in analoger Weise wie unter der Verfahrensvariante a) beschrieben verfahren wird.

In einer bevorzugten Ausführungsform erhält man Verbindungen VI, worin $X_4$ für Halogen steht und die gestrichelte Linie zum Ausdruck bringt, dass zwischen den Kohlenstoffatomen, welche die Substituenten $R_1$ und $X_4$ tragen, eine Einfachbindung vorliegt, und deren Salze durch Umsetzung einer Verbindung der Formel I, worin $R_2$ Hydroxy bedeutet und die gestrichelte Linie zum Ausdruck bringt, dass zwischen den Kohlenstoffatomen welche die Substituenten $R_1$ und $R_2$ tragen, eine Einfachbindung vorliegt, oder eines Salzes davon mit einem Halogenierungsmittel, wie Phosphortri-oder -pentachlorid oder Thionylchlorid, wobei die entsprechenden Verbindungen I und ihre Salze beispielsweise in analoger Weise wie unter der Verfahrensvariante a) oder c) beschrieben erhalten werden können.

Variante f):

Das Anion $A^{\ominus}$ ist beispielsweise das Anion einer starken Protonsäure, z.B. ein Halogenidion, wie Chlorid-, Bromid- oder Iodidion, oder ein Sulfonation, wie ein gegebenenfalls substituiertes Niederalkan- oder Benzolsulfonation, z.B. das Methansulfonat-, Aethansulfonat- oder p-Bromphenylsulfonat- oder p-Toluolsulfonation. Geschütztes Hydroxy ist beispielsweise Silyloxy, wie Triniederalkylsilyloxy, z.B. Trimethylsilyloxy, kann aber auch Triphenylniederalkoxy, z.B. Trityloxy, sein. Geschütztes Amino ist beispielsweise Silylamino, wie Triniederalkylsilylamino, z.B. Trimethylsilylamino, kann aber auch Phenyl-, Diphenyl- oder Triphenylniederalkylamino, wie Benzylamino, Diphenylamino oder Tritylamino, sein. Veräthertes Hydroxymethyl ist beispielsweise

Niederalkoxymethyl, wie Methoxy- oder Aethoxymethyl, oder gegebenenfalls substituiertes Phenylniederalkoxymethyl, z.B. im Phenylteil substituiertes Phenyl-$C_1$-$C_4$-alkoxymethyl, wie Benzyloxy-, p-Chlorbenzyloxy-, 1-Phenyläthyloxy-oder 1-(p-Bromphenyl)-n-butyloxymethyl. Geschütztes Hydroxymethyl ist beispielsweise Silyloxymethyl, wie Triniederalkylsilyl-, z.B. Trimethylsilyloxymethyl, kann aber auch Triphenylniederalkoxy-, z.B. Trityloxymethyl, sein.

Besonders geeignet ist die Verfahrensvariante f) zur Herstellung einer Verbindung der Formel I oder eines Tautomeren und/oder Salzes davon, worin entweder $R_1$ Carboxy, Niederalkoxycarbonyl, amidiertes Carboxy oder Hydroxymethyl bedeutet und $R_2$ Wasserstoff, Hydroxy oder Amino ist oder worin $R_1$ Wasserstoff darstellt und $R_2$ für Carboxy, Niederalkoxycarbonyl, amidiertes Carboxy oder Hydroxymethyl steht.

Die Reduktion der überschüssigen Doppelbindungen erfolgt durch Behandeln mit einem geeigneten Reduktionsmittel, beispielsweise durch Hydrierung in Gegenwart eines Hydrierungskatalysators, durch Reduktion mit einem Hydrid-übertragenden Reagenz oder durch Reduktion mit einem metallischen Reduktionssystem aus Metall und Protonen-abspaltendem Mittel.

Als Hydrierungskatalysatoren kommen z.B. Elemente der VIII. Nebengruppe des Periodensystems der Elemente oder deren Derivate in Betracht, wie Palladium, Platin, Platinoxid, Ruthenium, Rhodium, Tris(triphenylphosphan)-rhodium-I-halogenid, z.B. -chlorid, oder Raney-Nickel, die gegebenenfalls auf einem Trägermaterial, wie Aktivkohle, einem Alkalimetallcarbonat bzw. -sulfat oder einem Kieselgel aufgezogen sind. Als Hydrid-übertragende Reagenzien kommen beispielsweise geeignete Leichtmetallhydride, insbesondere Alkalimetallaluminiumhydride bzw. -borhydride, wie Lithiumaluminiumhydrid, Lithiumtriäthylborhydrid, Natriumborhydrid, Natriumcyanoborhydrid, oder Zinnhydride, wie Triäthyl- oder Tributylzinnhydrid, oder Diboran in Frage. Der Metallbestandteil des metallischen Reduktionssystems ist beispielsweise ein unedles Metall, wie Alkali-oder Erdalkalimetall, z.B. Lithium, Natrium, Kalium, Magnesium oder Calcium, oder Uebergangsmetall, z.B. Zink, Zinn, Eisen oder Titan, während als Protonen-abspaltende Mittel z.B. Protonsäuren der vorstehend genannten Art, wie Salz- oder Essigsäure, Niederalkanole, wie Aethanol, und/oder Amine bzw. Ammoniak in Frage kommen. Solche Systeme sind beispielsweise Natrium/Ammoniak, Zink/Salz- oder Essigsäure oder Zink/Aethanol.

Falls $R_1''$ von $R_1$ und/oder $R_2''$ von $R_2$ verschieden ist, erfolgt die Ueberführung von $R_1''$ in $R_1$ und/oder $R_2''$ in $R_2$, d.h. die Abspaltung der Schutzgruppen und damit die Freisetzung der entsprechenden Reste $R_1$ und/oder $R_2$, in demselben oder in einem nachfolgenden Reaktionsschritt, wobei in üblicher Weise, z.B. wie im Anschluss an die Verfahrensvariante g) beschrieben, gearbeitet wird.

Die Herstellung von Ausgangsverbindungen der Formel VII erfolgt beispielsweise durch Umsetzung von Verbindungen der Formel

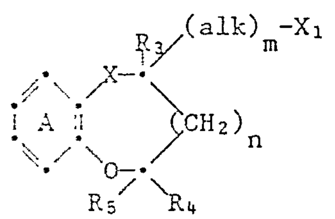

(IIa),

worin $X_1$ reaktionsfähiges verestertes Hydroxy, das in einfach negativ geladener Form dem Anion $A^{\ominus}$ entspricht, bedeutet, mit Verbindungen der Formel

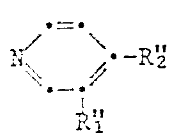

(VIIa)

oder einem Salz davon, wobei z.B. in analoger Weise wie unter der Verfahrensvariante a) beschrieben verfahren wird.

Variante g):

Abspaltbare Reste $Y_2$ in Verbindungen VIII sind beispielsweise reaktionsfähige veresterte Hydroxygruppen, wie mit einer starken anorganischen Säure oder organischen Sulfonsäure verestertes Hydroxy, beispielsweise Halogen, wie Chlor, Brom oder Iod, Sulfonyloxy, wie Hydroxysulfonyloxy, Halogensulfonyloxy, z.B. Fluorsulfonyloxy, gegebenenfalls, z.B. durch Halogen, substituiertes Niederalkansulfonyloxy, z.B. Methan- oder Trifluormethansulfonyloxy, Cycloalkansulfonyloxy, z.B. Cyclohexansulfonyloxy, oder gegebenenfalls, z.B. durch Niederalkyl oder Halogen, substituiertes Benzolsulfonyloxy, z.B. p-Bromphenyl- oder p-Toluolsulfonyloxy. oder verätherte Hydroxygruppen, beispielsweise Niederalkoxy oder gegebenenfalls substituiertes

Phenylniederalkoxy.

Die Cyclisierung kann beispielsweise in Gegenwart einer der unter der Variante a) genannten Basen, insbesondere in Gegenwart eines Alkalimetallniederalkanolats, z.B. mit Natriummethanolat oder -äthanolat, durchgeführt werden.

Die Ausgangsstoffe VIII werden beispielsweise erhalten, indem man eine Verbindung der Formel

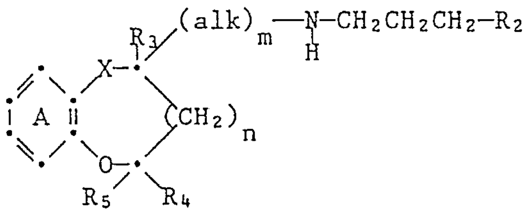

(IVh)

oder ein Salz davon mit einer Verbindung der Formel $Y_2$-$CH_2$-$CH(Y_2)$-$R_1$ (IVi) umsetzt.

In den Ausgangsstoffen der Formeln IIb, III, IIIa, IV, IVg, VII und VIIa kann eine Hydroxygruppe $R_2$ in verätherter bzw. eine Hydroxy- oder Amino-gruppe $R_2$ auch in intermediär geschützter Form vorliegen, ebenso wie eine Hydroxymethylgruppe $R_1$ bzw. $R_2$ in Verbindungen IIb, IVi, Vb, VI, VIa, VII, VIIa und VIII in verätherter oder intermediär geschützter Form vorliegen kann. Geschütztes Hydroxy ist beispielsweise Silyloxy, wie Tri niederalkylsilyloxy, z.B. Trimethylsilyloxy, kann aber auch Triphenylniederalkoxy, z.B. Trityloxy, sein. Geschütztes Amino ist beispielsweise Silylamino, wie Triniederalkylsilylamino, z.B. Trimethylsilylamino, kann aber auch Phenyl-, Diphenyl- oder Triphenylniederalkylamino, wie Benzylamino, Diphenylmethylamino oder Tritylamino, sein. Veräthertes Hydroxymethyl ist beispielsweise Niederalkoxymethyl, wie Methoxy-oder Aethoxymethyl, oder gegebenenfalls substituiertes Phenylniederalkoxymethyl, z.B. im Phenylteil substituiertes Phenyl-$C_1$-$C_4$-alkoxymethyl, wie Benzyloxy-, p-Chlorbenzyloxy-, 1-Phenyläthyloxy- oder 1-(p-Bromphenyl)-n-butyloxymethyl. Geschütztes Hydroxymethyl ist beispielsweise Silyloxymethyl, wie Triniederalkylsilyl-, z.B. Trimethylsilyloxymethyl, kann aber auch Triphenylniederalkoxy-, z.B. Trityloxymethyl, sein.

Die Freisetzung verätherter bzw. intermediär geschützter Reste $R_1$ bzw. $R_2$, d.h. Abspaltung der intermediären Schutzgruppen, erfolgt in üblicher Weise, beispielsweise durch Solvolyse, wie milde Hydrolyse, z.B. Behandlung mit Wasser unter neutralen oder schwach sauren Bedingungen, z.B. durch Einwirkung von verdünnt-wässrigen Mineral- oder Carbonsäuren, z.B. von verdünnter Salz- oder Essigsäure. In analoger Weise erfolgt die Freisetzung von intermediär geschützten Hydroxy- und Aminogruppen $R_2''$ sowie von verätherten oder geschützten Hydroxymethylgruppen $R_1''$ und $R_2''$ in Ausgangsstoffen der Formel VII bzw. VIIa.

Verfahrensgemäss oder anderweitig erhältliche Verbindungen der Formel I können in üblicher Weise in andere Verbindungen der Formel I überführt werden.

So kann man z.B. veresterte oder amidierte Carboxygruppen $R_1$ bzw. $R_2$ in üblicher Weise, beispielsweise in Gegenwart eines basischen oder sauren Hydrolysemittels, wie eines Alkalimetallhydroxides oder -carbonates, z.B. von Natriumhydroxid oder Kaliumcarbonat, oder einer Mineralsäure, z.B. von Salzsäure oder Schwefelsäure, zu Carboxy $R_1$ bzw. $R_2$ hydrolysieren. Veresterte Carboxygruppen $R_1$ bzw. $R_2$ können ferner durch Umesterung, d.h. Behandlung mit einem Alkohol in Gegenwart eines sauren oder basischen Solvolysemittels, wie einer Mineralsäure, z.B. von Schwefelsäure, bzw. eines entsprechenden Alkalimetallalkoholates oder eines Alkalimetallhydroxides, in andere veresterte Carboxygruppen $R_1$ bzw.

$R_2$ oder durch Umsetzung mit Ammoniak oder einem entsprechenden mindestens ein Wasserstoffatom aufweisenden Amin in amidiertes Carboxy $R_1$ bzw. $R_2$ überführt werden.

Freies Carboxy $R_1$ bzw. $R_2$ kann in üblicher Weise, beispielsweise durch Behandlung mit einem entsprechenden Alkohol in Gegenwart einer Mineralsäure, z.B. von Schwefelsäure, oder durch Ueberführung in ein Halogenid und anschliessende Umsetzung mit einem entsprechenden Alkohol, z.B. in Gegenwart von Pyridin oder Triäthylamin, oder durch Ueberführung in ein Alkalimetallsalz und anschliessende Umsetzung mit einem reaktionsfähigen Ester des entsprechenden Alkohols, wie einem entsprechenden Halogenid, in verestertes Carboxy $R_1$ bzw. $R_2$ überführt werden. Ebenso kann eine Carboxyverbindung unter Verwendung eines Dehydratisierungsmittels, wie N,N'-Dicyclohexylcarbodiimid, mit einem entsprechenden Alkohol verestert werden. Freies oder verestertes Carboxy $R_1$ bzw. $R_2$ kann auch durch Umsetzung mit Ammoniak oder einem mindestens ein Wasserstoffatom aufweisenden Amin und Dehydratisierung des intermediär gebildeten Ammoniumsalzes, z.B. durch Erhitzen oder mittels eines Dehydratisierungsmittels, wie N,N'-Dicyclohexylcarbodiimid, oder durch Ueberführung in das Halogenid und anschliessende Umsetzung mit Ammoniak oder einem mindestens ein Wasserstoffatom aufweisenden Amin in amidiertes Carboxy $R_1$ bzw. $R_2$ überführt werden.

Ferner kann man gegebenenfalls vorhandene Hydroxygruppen verestern, z.B. durch Behandeln mit einem Niederalkancarbonsäureanhydrid bzw. -halogenid in Niederalkanoyloxy überführen oder durch Umsetzung mit einem reaktionsfähigen Ester, insbesondere Brom- oder Chlorwasserstoffsäureester, eines Niederalkanols in entsprechendes veräthertes Hydroxy überführen. Umgekehrt kann man aus verestertem oder veräthertem Hydroxy, wie Niederalkanoyloxy oder Niederalkoxy, die Hydroxygruppe solvolytisch freisetzen, vorzugsweise

unter sauren Bedingungen. In analoger Weise kann man auch veräthertes oder acyliertes Hydroxy $R_2$ zu Hydroxy hydrolysieren.

Entsprechend kann man weiterhin Hydroxymethyl $R_1$ bzw. $R_2$ verestern, z.B. durch Behandeln mit einem Niederalkancarbonsäureanhydrid bzw. -halogenid in Niederalkanoyloxymethyl $R_1$ bzw. $R_2$ überführen. Umgekehrt kann man aus acyliertem Hydroxymethyl, z.B. Niederalkanoyloxymethyl, $R_1$ bzw. $R_2$ die Hydroxygruppe solvolytisch freisetzen, vorzugsweise unter sauren Bedingungen.

Weiterhin lässt sich Hydroxymethyl $R_1$ bzw. $R_2$ in üblicher Weise in Niederalkoxycarbonyl oder amidiertes Carboxy $R_1$ bzw. $R_2$ überführen, wobei z.B. in der Weise verfahren wird, dass zunächst Hydroxymethyl $R_1$ bzw. $R_2$ in üblicher Weise, z.B. in Gegenwart eines Oxidationsmittels, wie Kaliumpermanganat oder Kaliumdichromat, zu Carboxy oxidiert wird und anschliessend die Carboxygruppe in üblicher Weise, z.B. durch Behandlung mit einem entsprechenden Alkohol in Gegenwart einer Mineralsäure, z.B. von Schwefelsäure, oder durch Ueberführung in ein Halogenid und anschliessende Umsetzung mit einem entsprechenden Alkohol, z.B. in Gegenwart von Pyridin oder Triäthylamin, oder durch Ueberführung in ein Alkalimetallsalz und anschliessende Umsetzung mit einem reaktionsfähigen Ester des entsprechenden Alkohols, wie einem entsprechenden Halogenid, oder unter Verwendung eines Dehydratisierungsmittels, wie N,N'-Dicyclohexylcarbodiimid, mit einem entsprechenden Alkohol in Niederalkoxycarbonyl $R_1$ bzw. $R_2$ oder durch Umsetzung mit Ammoniak oder einem mindestens ein Wasserstoffatom aufweisenden Amin und Dehydratisierung des intermediär gebildeten Ammoniumsalzes, z.B. durch Erhitzen oder mittels eines Dehydratisierungsmittels, wie N,N'-Dicyclohexylcarbodiimid, oder durch Ueberführung in das Halogenid und anschliessende Umsetzung mit Ammoniak oder einem mindestens ein Wasserstoffatom aufweisenden Amin in amidiertes Carboxy $R_1$ bzw. $R_2$ überführt wird. Ebenso kann man acyliertes Hydroxymethyl $R_1$ bzw. $R_2$ in verestertes oder amidiertes Carboxy $R_1$ bzw. $R_2$ überführen, indem man zunächst die acylierte Hydroxymethylgruppe solvolytisch freisetzt, z.B. wie vorstehend beschrieben, und dann die erhaltene freie Hydroxymethylgruppe, wie vorstehend erläutert, in eine Carboxylgruppe und letztere weiter in eine veresterte oder amidierte Carboxylgruppe überführt. Umgekehrt lassen sich veresterte oder amidierte Carboxygruppen $R_1$ bzw. $R_2$ in gegebenenfalls acyliertes Hydroxymethyl $R_1$ bzw. $R_2$ überführen, indem man zunächst die veresterte oder amidierte Carboxygruppe $R_1$ bzw. $R_2$ in üblicher Weise, beispielsweise in Gegenwart eines basischen oder sauren Hydrolysemittels, wie eines Alkalimetallhydroxides oder -carbonates, z.B. von Natriumhydroxid oder Kaliumcarbonat, oder einer Mineralsäure, z.B. von Salzsäure oder Schwefelsäure, zu Carboxy hydrolysiert und anschliessend die erhaltene Carboxygruppe in üblicher Weise, z.B. in Gegenwart eines Reduktionsmittels, beispielsweise der vorstehend erwähnten Art, zu Hydroxymethyl $R_1$ bzw. $R_2$ reduziert, wobei gewünschtenfalls letzteres, z.B. wie vorstehend beschrieben, anschliessend noch in acyliertes Hydroxymethyl $R_1$ bzw. $R_2$ überführt werden kann.

In Verbindungen der Formel I, in denen die gestrichelte Linie zum Ausdruck bringt, dass zwischen den Kohlenstoffatomen, welche die Substituenten $R_1$ und $R_2$ tragen, eine Doppelbindung vorliegt, kann diese z.B. in an sich bekannter Weise mit Hilfe eines Reduktionsmittels, z.B. der unter Variante f) aufgeführten Art, zu einer Einfachbindung hydriert werden.

Ferner kann eine Verbindung der Formel I, in der die gestrichelte Linie zum Ausdruck bringt, dass zwischen den Kohlenstoffatomen, welche die Substituenten $R_1$ und $R_2$ tragen, eine Doppelbindung vorliegt, und $R_2$ Wasserstoff bedeutet, z.B. in an sich bekannter Weise durch Addition einer Verbindung $R_2$-H, in der $R_2$ eine gegebenenfalls verätherte oder acylierte Hydroxygruppe oder eine gegebenenfalls acylierte Aminogruppe darstellt, in eine entsprechende Piperidinverbindung überführt werden. Die Addition wird dabei insbesondere in Gegenwart einer geeigneten Base, z.B. der unter Variante a) aufgeführten Art, durchgeführt.

Verbindungen der Formel I, in denen die gestrichelte Linie zum Ausdruck bringt, dass zwischen den Kohlenstoffatomen, welche die Substituenten $R_1$ und $R_2$ tragen, eine Einfachbindung vorliegt, können umgekehrt z.B. durch Eliminierung einer Verbindung $R_2$-H, in der $R_2$ eine gegebenenfalls verätherte oder acylierte Hydroxygruppe oder eine gegebenenfalls acylierte Aminogruppe darstellt, in an sich bekannter Weise in entsprechende Tetrahydro-Pyridin-Verbindungen, in denen $R_2$ Wasserstoff darstellt, überführt werden. Für eine Eliminierung schlechter geeignete Abgangsgruppen $R_2$, z.B. Hydroxy, können dabei zuvor, beispielsweise in situ, in besser geeignete Abgangsgruppen $R_2$, z.B. Niederalkansulfonyloxy, wie Methansulfonyloxy, oder Halogen, wie Chlor, Brom oder Iod, umgewandelt werden. Die Eliminierung erfolgt dabei insbesondere in Gegenwart einer geeigneten Base, z.B. der unter Variante a) aufgeführten Art.

Salze von Verbindungen der Formel I bzw. von deren Tautomeren können in an sich bekannter Weise hergestellt werden. So erhält man beispielsweise Säureadditionssalze von Verbindungen der Formel I durch Behandeln mit einer Säure oder einem geeigneten Ionenaustauscherreagens. Salze können in üblicher Weise in die freien Verbindungen der Formel I überführt werden, Säureadditionssalze z.B. durch Behandeln mit einem geeigneten basischen Mittel.

Je nach Verfahrensweise bzw. Reaktionsbedingungen können die Verbindungen der Formel I mit salzbildenden, insbesondere basischen, Eigenschaften in freier Form oder in Form von Salzen erhalten werden.

Infolge der engen Beziehung zwischen der neuen Verbindung der Formel I in freier Form und in Form ihrer Salze sind im vorausgegangenen und nachfolgend unter der freien Verbindung der Formel I oder ihren Salzen sinn- und zweckgemäss gegebenenfalls auch die entsprechenden Salze bzw. die freie Verbindung der Formel I zu verstehen.

Die neuen verbindungen der Formel I einschliesslich ihrer Salze von salzbildenden Verbindungen können auch in Form ihrer Hydrate erhalten werden oder andere, beispielsweise zur Kristallisation von in fester Form

vorliegenden Verbindungen verwendete, Lösungsmittel einschliessen.

Die neuen Verbindungen der Formel I können, je nach Wahl der Ausgangsstoffe und Arbeitsweisen, in Form eines der möglichen Isomeren oder als Gemisch derselben vorliegen. Dabei sind, in Abhängigkeit von der Molekülsymmetrie, z.B. je nach Anzahl, absoluter und relativer Konfiguration der Chiralitätszentren, wie asymmetrischen C-Atome, als reine Isomere z.B. reine Enantiomere und/oder reine Diastereomere, wie reine cis/transIsomere oder meso-Verbindungen, erhältlich. Entsprechend können als Isomerengemische z.B. Enantiomerengemische, wie Racemate, Diastereomerengemische oder Racematgemische vorliegen.

Erhaltene Diastereomerengemische und Racematgemische können aufgrund der physikalisch-chemischen Unterschiede der Bestandteile in bekannter Weise in die reinen Diastereomeren oder Racemate aufgetrennt werden, beispielsweise durch fraktionierte Kristallisation.

Erhaltene Enantiomerengemische, wie Racemate, lassen sich nach bekannten Methoden in die Enantiomeren zerlegen, beispielsweise durch Umkristallisation aus einem optisch aktiven Lösungsmittel, Chromatographie an chiralen Adsorbentien, mit Hilfe von geeigneten Mikroorganismen, durch Spaltung mit spezifischen, immobilisierten Enzymen, über die Bildung von Einschlussverbindungen, z.B. unter Verwendung chiraler Kronenäther, wobei nur ein Enantiomeres komplexiert wird, oder durch Ueberführung in diastereomere Salze, z.B. durch Umsetzung eines basischen Endstoffracemats mit einer optisch aktiven Säure, wie Carbonsäure, z.B. Wein-oder Aepfelsäure, oder Sulfonsäure, z.B. Camphersulfonsäure, und Trennung des auf diese Weise erhaltenen Diastereomerengemisches, z.B. auf Grund ihrer verschiedenen Löslichkeiten, in die Diastereomeren, aus denen das gewünschte Enantiomere durch Einwirkung geeigneter Mittel freigesetzt werden kann. Vorteilhaft isoliert man das wirksamere Enantiomere.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Derivates bzw. Salzes und/oder seiner Racemate bzw. Enantiomeren verwendet oder insbesondere unter den Reaktionsbedingungen bildet.

Beim Verfahren der vorliegenden Erfindung werden vorzugsweise solche Ausgangsstoffe verwendet, welche zu den eingangs als besonders wertvoll geschilderten Verbindungen der Formel I führen. Neue Ausgangsstoffe, die speziell für die Herstellung der Verbindungen der Formel I entwickelt wurden, ihre Verwendung und Verfahren zu ihrer Herstellung bilden ebenfalls einen Gegenstand der Erfindung, wobei die Variablen $R_1$, $R_2$, $R_3$, $R_4$, $R_5$, X, m, n und alk sowie die Substituenten des Ringes A und die gestrichelte Linie die für die jeweils bevorzugten Verbindungsgruppen der Formel I angegebenen Bedeutungen haben.

In diesem Zusammenhang sind insbesondere Verbindungen der Formel

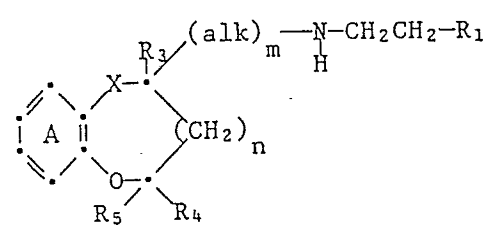

(IVc)

und deren Salze zu erwähnen. Diese besitzen ebenfalls nootrope Eigenschaften in vergleichbarer Wirkungsstärke wie die entsprechenden Verbindungen der Formel I bzw. I′ und können ebenfalls als nootrope Arzneimittelwirkstoffe Verwendung finden.

Die Erfindung betrifft dementsprechend ebenfalls Verbindungen der Formel IVc, worin $R_1$ Carboxy, Niederalkoxycarbonyl, amidiertes Carboxy oder gegebenenfalls acyliertes Hydroxymethyl bedeutet, $R_3$ Wasserstoff oder Niederalkyl darstellt, $R_4$ Niederalkyl ist, $R_5$ Niederalkyl darstellt, alk Niederalkylen oder Niederalkyliden bedeutet, der Ring A unsubstituiert oder ein- oder mehrfach durch Hydroxy, Niederalkoxy, Niederalkanoyloxy, Cyano, Halogen, Niederalkyl und/oder Trifluormethyl substituiert ist, m 0 oder 1 bedeutet und worin entweder X ein Sauerstoffatom oder eine Methylengruppe bedeutet und n für 0 steht oder X für eine direkte Bindung steht und n für 1 steht, und ihre Salze, die Verwendung der genannten Verbindungen der Formel IVc, ein Verfahren zu ihrer Herstellung und pharmazeutische Zusammensetzungen, enthaltend eine Verbindung der Formel IVc oder ein pharmazeutisch verwendbares Salz davon.

Die Variablen der Formel IVc haben dabei beispielsweise die unter Formel I angegebenen bevorzugten Bedeutungen.

Die Erfindung betrifft diesbezüglich in erster Linie Verbindungen der Formel IVc, worin $R_1$ Carboxy, Niederalkoxycarbonyl, Carbamyl, N-Niederalkylcarbamyl, N,N-Diniederalkylcarbamyl, N,N-Niederalkylencarbamyl, N,N-(Aza)-, N,N-(Oxa)- oder N,N-(Thia)niederalkylencarbamyl, Hydroxymethyl, Niederalkanoyloxymethyl, Niederalkansulfonyloxymethyl, Benzoyloxymethyl oder Pyridoyloxymethyl bedeutet, $R_3$ Wasserstoff oder Niederalkyl darstellt, $R_4$ Niederalkyl ist, $R_5$ Niederalkyl darstellt, alk Niederalkylen, welches das Ringsystem mit der in Formel IVc einge zeichneten NH-Gruppe durch bis und mit 3 C-Atome verknüpft, oder Niederalkyliden darstellt, der Ring A unsubstituiert ist oder ein-, zwei- oder mehrfach durch Hydroxy, Niederalkoxy, Niederalkanoyloxy, Cyano, Halogen Niederalkyl und/oder Trifluormethyl substituiert ist, m 0 oder 1 bedeutet und worin entweder X ein Sauerstoffatom oder eine Methylengruppe bedeutet und n für 0 steht oder X für eine

direkte Bindung steht und n für 1 steht, und ihre Salze.

Die Erfindung betrifft diesbezüglich vor allem Verbindungen der Formel IVc, worin $R_1$ $C_1$-$C_4$-Alkoxycarbonyl, wie Methoxycarbonyl oder Aethoxycarbonyl, Carbamyl, N,N-Niederalkylencarbamyl, wie Piperidinocarbonyl, N,N-(Aza)niederalkylencarbamyl, wie Piperazinocarbonyl, N,N-(Oxa)niederalkylencarbamyl, wie Morpholinocarbonyl, oder N,N-(Thia)niederalkylencarbamyl, wie Thiomorpholinocarbonyl, bedeutet, $R_3$ Wasserstoff ist, $R_4$ $C_1$-$C_4$-Alkyl, wie Methyl, bedeutet, $R_5$ $C_1$-$C_4$-Alkyl, wie Methyl, darstellt, alk für $C_1$-$C_4$-Alkylen, welches das Ringsystem mit der in Formel IVc eingezeichneten NH-Gruppe durch bis und mit 3 C-Atome verknüpft, wie Methylen oder Aethylen, steht, der Ring A unsubstituiert oder, insbesondere in 6-Stellung, durch $C_1$-$C_4$-Alkoxy, wie Methoxy, $C_1$-$C_4$-Alkyl, wie Methyl, Halogen mit einer Atomnummer bis und mit 35, wie Fluor oder Chlor, Cyano oder Trifluormethyl substituiert ist, m für 0 oder 1 steht, X ein Sauerstoffatom oder eine Methylengruppe bedeutet und n für 0 steht, und ihre Salze.

Die Erfindung betrifft diesbezüglich insbesondere Verbindungen der Formel IVc, worin $R_1$ $C_1$-$C_4$-Alkoxycarbonyl, wie Methoxycarbonyl oder Aethoxycarbonyl, oder Carbamyl bedeutet, $R_3$ Wasserstoff ist, $R_4$ $C_1$-$C_4$-Alkyl, wie Methyl, bedeutet, $R_5$ $C_1$-$C_4$-Alkyl, wie Methyl, darstellt, alk für $C_1$-$C_4$-Alkylen, welches das Ringsystem mit der in Formel IVc eingezeichneten NH-Gruppe durch bis und mit 3 C-Atome verknüpft, wie Methylen oder Aethylen, steht, der Ring A unsubstituiert oder, insbesondere in 6-Stellung, einfach durch Halogen mit einer Atomnummer bis und mit 35, wie Fluor oder Chlor, substituiert ist, m für 1 steht, X eine Methylengruppe darstellt und n für 0 steht, und ihre Salze.

Die Erfindung betrifft diesbezüglich in allererster Linie Verbindungen der Formel IVc, worin $R_1$ $C_1$-$C_4$-Alkoxycarbonyl, wie Methoxycarbonyl, oder Carbamyl bedeutet, $R_3$ Wasserstoff ist, $R_4$ $C_1$-$C_4$-Alkyl, wie Methyl, bedeutet, $R_5$ $C_1$-$C_4$-Alkyl, wie Methyl, darstellt, alk für Aethylen steht, der Ring A unsubstituiert ist, m für 1 steht, X eine Methylengruppe darstellt und n für 0 steht, und ihre Salze.

Die Erfindung betrifft diesbezüglich namentlich die in den Beispielen genannten neuen Verbindungen der Formel IVc und ihre Salze sowie Verfahren zu ihrer Herstellung.

Ebenfalls Gegenstand der vorliegenden Erfindung ist ein Verfahren zur Herstellung von Verbindungen der Formel IVc oder ihrer Salze, z.B. dadurch gekennzeichnet, dass man

h) Verbindungen der Formeln

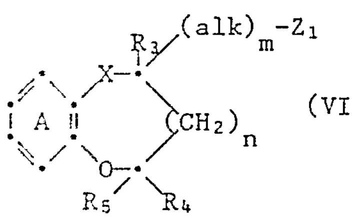

(VIIIa)

und $Z_2$-$CH(Z_3)$-$R_1$ (VIIIb),

worin einer der Reste $Z_1$ und $Z_2$ reaktionsfähiges verestertes Hydroxy, der andere Amino und $Z_3$ Wasserstoff bedeutet oder $Z_1$ Amino ist und $Z_2$ sowie $Z_3$ gemeinsam eine zusätzliche Bindung darstellen, oder gegebenenfalls Salze dieser Verbindungen untereinander umsetzt oder

i) in einer Verbindung der Formel

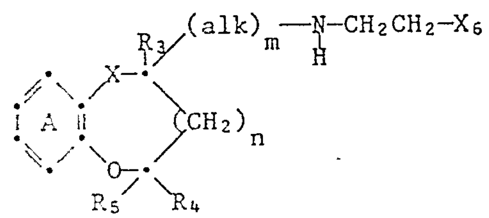

(IX),

worin $X_6$ einen in $R_1$ überführbaren Rest bedeutet, oder einem Salz davon $X_6$ in $R_1$ überführt und jeweils eine gegebenenfalls vorhandene Schutzgruppe abspaltet und jeweils gewünschtenfalls eine verfahrensgemäss oder auf andere Weise erhältliche Verbindung der Formel IVc in eine andere Verbindung der Formel IVc überführt, jeweils ein verfahrensgemäss erhältliches Isomerengemisch in die Komponenten auftrennt, jeweils ein verfahrensgemäss erhältliches Enantiomeren- bzw. Diastereomerengemisch in die Enantiomeren bzw. Diastereomeren aufspaltet und/oder jeweils eine verfahrensgemäss erhältliche freie Verbindung der Formel IVc in ein Salz überführt oder jeweils ein verfahrensgemäss erhältliches Salz in die freie Verbindung der Formel IVc oder in ein anderes Salz umwandelt.

Variante h):

Reaktionsfähiges verestertes Hydroxy $Z_1$ bzw. $Z_2$ bedeutet insbesondere mit einer starken anorganischen Säure oder organischen Sulfonsäure verestertes Hydroxy, beispielsweise Halogen, wie Chlor, Brom oder Iod, Sulfonyloxy, wie Hydroxysulfonyloxy, Halogensulfonyloxy, z.B. Fluorsulfonyloxy, gegebenenfalls, z.B. durch Halogen, substituiertes Niederalkansulfonyloxy, z.B. Methan- oder Trifluormethansulfonyloxy, Cycloalkansulfonyloxy, z.B. Cyclohexansulfonyloxy, oder gegebenenfalls, z.B. durch Niederalkyl oder Halogen, substituiertes Benzolsulfonyloxy, z.B. p-Bromphenyl- oder p-Toluolsulfonyloxy.

Die Umsetzung wird in diesem Fall insbesondere in Gegenwart eines Kondensationsmittels, wie einer geeigneten Base, durchgeführt. Als Basen kommen beispielsweise Alkalimetallhydroxide, -hydride, -amide, -alkanolate, -carbonate, -triphenylmethylide, -diniederalkylamide, -aminoniederalkylamide oder -niederalkylsilylamide, Naphthalinamine, Niederalkylamine, basische Heterocyclen, Ammoniumhydroxide sowie carbocyclische Amine in Frage. Beispielhaft seien Natriumhydroxid, -hydrid, -amid, -äthylat, Kalium-tert-butylat, -carbonat, Lithiumtriphenylmethylid, Lithium-diisopropylamid, Kalium-3-(aminopropyl)-amid, -bis-(trimethylsilyl)-amid, Dimethylaminonaphthalin, Di- oder Triäthylamin, Pyridin, Benzyl-trimethyl-ammoniumhydroxid, 1,5-Diaza-bicyclo[4.3.0]non-5-en (DBN) sowie 1,5-Diaza-bicyclo[5.4.0]undec-5-en (DBU) genannt. Die Umsetzung von Aminen VIIIa ($Z_1$ = Amino) mit Acrylsäureverbindungen VIIIb ($Z_2 + Z_3$ = Bindung) erfolgt beispielsweise unter Erwärmen, z.B. auf etwa 60-120°C.

Das Ausgangsmaterial der Formeln VIIIa und VIIIb ist bekannt oder kann in Analogie zu bekannten Methoden hergestellt werden.

Variante i):

Ein in $R_1$ überführbarer Rest $X_6$ ist beispielsweise von $R_1$ verschiedenes funktionell abgewandeltes Carboxy, wie Cyano, anhydridisiertes Carboxy, gegebenenfalls substituiertes Amidino, gegebenenfalls verestertes oder anhydridisiertes Carboximidoyl, von verestertem oder amidiertem Carboxy $R_1$ verschiedenes verestertes oder amidiertes Carboxy, Triniederalkoxy-oder Trihalogenmethyl.

Anhydridisiertes Carboxy ist beispielsweise mit einer Mineralsäure, wie Halogenwasserstoffsäure, oder mit einer Carbonsäure, wie einer gegebenenfalls substituierten Niederalkan- bzw. Benzoesäure, oder einem Kohlensäurehalogenid-niederalkylhalbester anhydridisiertes Carboxy. Als Beispiele seien Halogencarbonyl, wie Chlorcarbonyl, Niederalkanoyloxycarbonyl, wie Acetyloxycarbonyl, oder Niederalkoxycarbonyloxycarbonyl, wie Aethoxycarbonyloxycarbonyl, genannt.

Substituiertes Amidino ist beispielsweise mit einem aliphatischen Rest, z.B. Niederalkyl, substituiertes Amidino, wie Niederalkylamidino, z.B. Aethylamidino.

Unter verestertem oder anhydridisiertem Carboximidoyl ist z.B. Alkoxybzw. Halogencarboximidoyl, beispielsweise Niederalkoxy-, wie Aethoxy-, bzw. Chlorcarboximidoyl, zu verstehen.

Triniederalkoxy- bzw. Trihalogenmethyl ist z.B. Trimethoxymethyl bzw. Trichlormethyl.

$X_6$ kann beispielsweise durch Solvolyse in $R_1$ überführt werden. Solvolysemittel sind beispielsweise Wasser, dem gewünschten veresterten Carboxy $R_1$ entsprechende Niederalkanole, Ammoniak oder der gewünschten amidierten Carboxygruppe $R_1$ entsprechende Amine. Die Behandlung mit einem entsprechenden Solvolysemittel wird gegebenenfalls in Gegenwart einer Säure oder Base durchgeführt. Als Säuren kommen beispielsweise anorganische oder organische Protonsäuren, wie Mineralsäuren, z.B. Schwefelsäure oder Halogenwasserstoffsäuren, beispielsweise Chlorwasserstoffsäure, wie Sulfonsäuren, z.B. Niederalkan- oder gegebenenfalls substituierte Benzolsulfonsäure, beispielsweise Methan- oder p-Toluolsulfonsäure, oder wie Carbonsäuren, z.B. Niederalkancarbonsäuren, beispielsweise Essigsäure, in Frage, während als Basen beispielsweise die unter Variante h) genannten verwendet werden können, insbesondere Natrium- oder Kaliumhydroxid.

Bei der Solvolyse wird die Cyanogruppe, anhydridisiertes Carboxy, gegebenenfalls substituiertes Amidino, gegebenenfalls verestertes oder anhydridisiertes Carboximidoyl, von verestertem oder amidiertem Carboxy $R_1$ verschiedenes verestertes oder amidiertes Carboxy, Triniederalkoxyoder Trihalogenmethyl zu Carboxy hydrolysiert. Dabei können gegebenenfalls am Ring A befindliche Niederalkanoyloxyreste im Verlauf der Hydrolyse zu Hydroxy hydrolysiert werden.

Cyano, anhydridisiertes Carboxy, von verestertem oder amidiertem Carboxy $R_1$ verschiedenes verestertes oder amidiertes Carboxy werden beispielsweise mit einem geeigneten Niederalkanol zu verestertem Carboxy $R_1$ alkoholisiert und Cyano und anhydridisiertes Carboxy beispielsweise mit Ammoniak oder einem dem amidierten Carboxy $R_1$ entsprechenden Amin ammono-bzw. aminolysiert.

Das Ausgangsmaterial der Formel IX kann z.B. durch Umsetzung von Verbindungen der Formeln

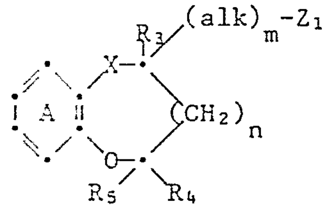

(VIIIa)

und $Z_2$-$CH_2$-$CH(Z_3)$-$X_6$ (IXb),

worin einer der Reste $Z_1$ und $Z_2$ reaktionsfähiges verestertes Hydroxy, der andere Amino und $Z_3$ Wasserstoff bedeutet oder $Z_1$ Amino ist und $Z_2$ sowie $Z_3$ gemeinsam eine zusätzliche Bindung darstellen, oder gegebenenfalls Salzen dieser Verbindungen erhalten werden, wobei in analoger Weise wie unter der Verfahrensvariante h) beschrieben, beispielsweise in Gegenwart eines basischen Mittels, gearbeitet wird. Reaktionsfähiges verestertes Hydroxy $Z_1$ bzw. $Z_2$ hat dabei beispielsweise eine der unter der Verfahrensvariante h) angegebenen Bedeutungen.

In den Ausgangsstoffen der Formel VIIIb kann eine Hydroxymethylgruppe $R_1$ in verätherter oder intermediär geschützter Form vorliegen. Veräthertes Hydroxymethyl ist beispielsweise Niederalkoxymethyl, wie Methoxy- oder Aethoxymethyl, oder gegebenenfalls substituiertes Phenylniederalkoxymethyl, z.B. im Phenylteil substituiertes Phenyl-$C_1$-$C_4$-alkoxymethyl, wie Benzyloxy-, p-Chlorbenzyloxy-, 1-Phenyläthyloxy- oder 1-(p-Bromphenyl)-n-butyloxymethyl. Geschütztes Hydroxymethyl ist beispielsweise Silyloxymethyl, wie Triniederalkylsilyl-, z.B. Trimethylsilyloxymethyl, kann aber auch Triphenylniederalkoxy-, z.B. Trityloxymethyl, sein.

Die Freisetzung intermediär geschützter Reste $R_1$, d.h. Abspaltung der intermediären Schutzgruppen, erfolgt in üblicher Weise, beispielsweise durch Solvolyse, wie milde Hydrolyse, z.B. Behandlung mit Wasser unter neutralen oder schwach sauren Bedingungen, z.B. durch Einwirkung von verdünnt-wässrigen Mineral- oder Carbonsäuren z.B. von verdünnter Salz- oder Essigsäure.

An verfahrensgemäss oder anderweitig erhältlichen Verbindungen IVc gewünschtenfalls vorzunehmende Nachoperationen sind insbesondere analoge Umwandlungsreaktionen von $R_1$ und von Substituenten des Ringes A, Enantiomeren- sowie Diastereomerentrennungen und gegenseitige Umwandlungen von Salzen und freien Verbindungen der Formel IVc wie für die Verbindungen der Formel I angegeben, die auch analog durchgeführt werden.

Die Erfindung betrifft ebenfalls die Verwendung von Verbindungen der Formel I bzw. IVc, gebenenfalls deren Tautomeren und/oder von pharmazeutisch verwendbaren Salzen von solchen Verbindungen mit salzbildenden Eigenschaften, insbesondere als pharmakologische, in erster Linie nootrop wirksame, Wirksubstanzen. Dabei kann man sie, vorzugsweise in Form von pharmazeutisch verwendbaren Zubereitungen, in einem Verfahren zur prophylaktischen und/oder therapeutischen Behandlung des tierischen oder menschlichen Körpers, insbesondere als Nootropika, z.B. zur Behandlung von cerebralen Insuffizienzerscheinungen, insbesondere Gedächtnisstörungen, anwenden.

Die Erfindung betrifft gleichfalls pharmazeutische Präparate, die eine Verbindung der Formel I bzw. IVc oder gegebenenfalls ein Tautomeres und/oder pharmazeutisch verwendbares Salz davon als Wirkstoff enthalten, sowie Verfahren zu ihrer Herstellung.

Bei den erfindungsgemässen pharmazeutischen Präparaten, welche eine Verbindung der Formel I bzw. IVc oder gegebenenfalls ein Tautomeres und/oder pharmazeutisch verwendbares Salz davon enthalten, handelt es sich um solche zur enteralen, wie oralen, ferner rektalen, und parenteralen Verabreichung an Warmblüter, wobei der pharmakologische Wirkstoff allein oder zusammen mit üblichen pharmazeutischen Hilfsstoffen enthalten ist.

Die neuen pharmazeutischen Präparate enthalten z.B. von etwa 10 % bis etwa 80 %, vorzugsweise von etwa 20 % bis etwa 60 %, des Wirkstoffs. Erfindungsgemässe pharmazeutische Präparate zur enteralen bzw. parenteralen Verabreichung sind z.B. solche in Dosiseinheitsformen, wie Dragées, Tabletten, Kapseln oder Suppositorien, ferner Ampullen. Diese werden in an sich bekannter Weise, z.B. mittels konventioneller Misch-, Granulier-, Dragier-, Lösungs- oder Lyophilisierungsverfahren hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen, zu Tabletten oder Dragée-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphat, z.B. Tricalciumphosphat oder Calciumhydrogenphosphat, ferner Bindemittel, wie Stärkekleister, unter Verwendung z.B. von Mais-, Weizen-, Reis- oder Kartoffelstärke, Gelatine, Tragaganth, Methylcellulose und/oder Polyvinylpyrrolidon, wenn erwünscht, Sprengmittel, wie die obengenannten Stärken, ferner Carboxymethylstärke, quervernetztes Polyvinylpyrrolidon, Agar, Alginsäure oder ein Salz davon, wie Natriumalginat. Hilfsmittel sind in erster Linie Fliessregulier- und Schmiermittel, z.B. Kieselsäure, Talk, Stearinsäure oder Salze davon, wie Magnesium- oder Calciumstearat, und/oder Polyäthylenglykol. Dragée-Kerne werden mit geeigneten, gegebenenfalls Magensaft-resistenten Ueberzügen versehen, wobei man u.a. konzentrierte Zuckerlösungen, welche gegebenfalls arabischen Gummi, Talk, Polyvinylpyrrolidon, Polyäthylenglykol und/oder Titandioxid enthalten, Lacklösungen in geeigneten organischen Lösungsmitteln oder Lösungsmittelgemischen oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbitol. Die Steckkapseln können den Wirkstoff in Form eines Granulats, z.B. im Gemisch mit Füllstoffen, wie Lactose, Bindemitteln, wie Stärken, und/oder Gleitmitteln, wie Talk oder Magnesiumstearat, und gegebenenfalls von Stabilisatoren, enthalten. In weichen Kapseln ist der Wirkstoff vorzugsweise in geeigneten Flüssigkeiten, wie fetten Oelen, Paraffinöl oder flüssigen Polyäthylenglykolen, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyäthylenglykole oder höhere Alkanole. Ferner können auch Gelatine-Rektalkapseln verwendet werden, die eine Kombination des Wirkstoffs mit einem Grundmassenstoff enthalten. Als Grundmassenstoffe kommen z.B. flüssige Triglyceride, Polyäthylenglykole oder Paraffinkohlenwasserstoffe in Frage.

Zur parenteralen Verabreichung eignen sich in erster Linie wässrige Lösungen eines Wirkstoffs in wasserlöslicher Form, z.B. eines wasserlöslichen Salzes, ferner Suspensionen des Wirkstoffs, wie entsprechende ölige Injektionssuspensionen, wobei man geeignete lipophile Lösungsmittel oder Vehikel, wie fette Oele, z.B. Sesamöl, oder synthetische Fettsäure ester, z.B. Aethyloleat oder Triglyceride, verwendet oder wässerige Injektionssuspensionen, welche viskositätserhöhende Stoffe, z.B. Natriumcarboxymethylcellulose, Sorbit und/oder Dextran, und gegebenenfalls auch Stabilisatoren enthalten.

Die Dosierung des Wirkstoffs kann von verschiedenen Faktoren, wie Applikationsweise, Warmblüter-Spezies, Alter und/oder individuellem Zustand abhängen. Im Normalfall ist für einen etwa 75 kg schweren Warmblüter bei oraler Applikation eine ungefähre Tagesdosis von etwa 20 bis etwa 500 mg, insbesondere von etwa 25 bis etwa 250 mg, vorteilhaft in mehreren gleichen Teildosen, zu veranschlagen.

Die nachfolgenden Beispiele illustrieren die oben beschriebene Erfindung; sie sollen jedoch diese in ihrem Umfang in keiner Weise einschränken. Temperaturen sind in Celsiusgraden angegeben.

Infolge der engen Beziehung zwischen einer Verbindung der Formel I und der entsprechenden tautomeren Verbindung der Formel I′ ist in den Beispielen unter einer Verbindung der Formel I sinn- und zweckgemäss gegebenenfalls auch die tautomere Verbindung der Formel I′ zu verstehen. Analoges gilt für eine Verbindung der Formel I′ sowie für Salze von Verbindungen der Formeln I und I′.

Beispiel 1:

Eine Lösung von 9 g (25 mmol) 2,2-Dimethyl-3-[2-(4-toluolsulfonyloxy)äthyl]-chroman in 100 ml N,N-Dimethylformamid wird zuerst mit 5,55 g (25 mmol) 1,2,5,6-Tetrahydro-pyridin-3-carbonsäuremethylester-hydrobromid (Guvacolin-hydrobromid) und anschliessend mit 11,31 g (87,5 mmol) N-Aethyl-N,N-diisopropylamin versetzt. Die Lösung wird 15 Stunden bei 60° gerührt und anschliessend im Hochvakuum eingedampft. Der Rückstand wird mit Wasser versetzt und mit Diäthyläther extrahiert. Die vereinigten organischen Phasen werden mit Wasser gewaschen und mit 2N-Salzsäure extrahiert. Die vereinigten salzsauren Extrakte werden unter Kühlung mit Natronlauge (30 %) alkalisch gestellt und mit Dichlormethan extrahiert. Die vereinigten Dichlormethan-Phasen werden über Natriumsulfat getrocknet und im Vakuum eingedampft. Man erhält 5,52 g (67 % d.Th.) 1-[2-(2,2-Dimethylchroman-3-yl)äthyl]-1,2,5,6-tetrahydro-pyridi-3-carbonsäuremethylester als hellgelbes Oel. Das hieraus mit Salzsäure in Diäthyläther hergestellte 1-[2-(2,2-Dimethylchroman--3-yl)äthyl]-1,2,5,6-tetrahydro-pyridin-3-carbonsäuremethylester-hydrochlorid kristallisiert aus Methanol/Diäthyläther und schmilzt bei 196-197°.

Das 2,2-Dimethyl-3-[2-(4-toluolsulfonyloxy)äthyl]-chroman lässt sich z.B. folgendermassen erhalten:

25 g (0,6 mol) Natriumhydrid-Dispersion (57 % in Mineralöl) werden durch mehrmaliges Waschen mit n-Hexan weitgehend vom Mineralöl befreit und danach mit 500 ml getrocknetem Tetrahydrofuran überschichtet. Unter Stickstoff und Kühlung in einem Eisbad werden innert 1 Stunde 134 g (0,6 mol) Phosphonoessigsäuretriäthylester der Formel $(H_5C_2O)_2P(=O)CH_2C(=O)OC_2H_5$ zugetropft. Man lässt eine weitere Stunde bei 0° nachrühren. Zu der homogenen Lösung wird sodann innert 30 Minuten eine Lösung von 88,57 g (0,5 mol) 2,2-Dimethyl-3-oxo-chroman [F. Camps et al., J. Heterocyclic Chem. 22, 1421 (1985)] in 300 ml Tetrahydrofuran zugetropft. Man rührt über Nacht bei Raumtemperatur nach. Dann wird die Lösung im vakuum auf etwa 350 ml konzentriert und auf 3 l eiskalte Phophatpufferlösung (pH = 6) gegossen. Das Gemisch wird mit Diäthyläther extrahiert. Die mit Wasser gewaschenen und über Natriumsulfat getrockneten vereinigten organischen Phasen werden im Vakuum eingedampft. Der gelbe Rückstand wird an 4 kg Kieselgel (0,040-0,063 mm) mit Toluol als Laufmittel chromatographiert. Man erhält so 60,3 g (49 % d.Th.) 3-Aethoxycarbonylmethyl-2,2-dimethyl-2H-chromen als hellgelbes Oel.

Eine Lösung von 59,1 g (0,24 mol) 3-Aethoxycarbonylmethyl-2,2-dimethyl-2H-chromen in 400 ml absolutem Aethanol wird mit 3 g Palladium auf Kohle (5 %) versetzt und in einer PARR-Apparatur bei Raumtemperatur

während 2 Stunden hydriert. Das Reaktionsgemisch wird sodann über Diatomeenerde filtriert. Das Filtrat wird zur Trockne eingedampft. Der ölige Rückstand besteht aus reinem 3-Aethoxycarbonylmethyl-2,2-dimethyl-chroman.

Zu einer Suspension von 7,6 g (0,2 mol) Lithiumaluminiumhydrid in 200 ml absolutem Diäthyläther wird unter Eiskühlung eine Lösung von 50 g (0,2 mol) 3-Aethoxycarbonylmethyl-2,2-dimethyl-chroman in 250 ml absolutem Diäthyläther innert 1 Stunde zugetropft. Das Reaktionsgemisch wird 3 Stunden bei Raumtemperatur nachgerührt und dann vorsichtig mit 7,5 ml Wasser, 7,5 ml Natronlauge (15 %) und 23 ml Wasser zersetzt. Der ausgefallene Niederschlag wird abfiltriert. Das über Natriumsulfat getrocknete Filtrat wird im Vakuum eingedampft. Man erhält 39,5 g (95 % d.Th.) 2,2-Dimethyl-3-(2-hydroxyäthyl)-chroman in Form eines farblosen Oels.

Eine Lösung von 37,1 g (0,18 mol) 2,2-Dimethyl-3-(2-hydroxyäthyl)-chroman in 150 ml Pyridin wird bei Raumtemperatur unter Rühren mit 38 g (0,2 mol) 4-Toluolsulfonylchlorid versetzt, wobei die leicht exotherme Reaktion mit einem Eisbad bei Raumtemperatur gehalten wird. Man lässt 4 Stunden bei Raumtemperatur nachrühren. Das Reaktionsgemisch wird sodann auf Eiswasser gegossen und mit Diäthyläther extrahiert. Die vereinigten ätherischen Phasen werden dreimal mit je 150 ml Citronensäurelösung (5 %) und dreimal mit je 150 ml Wasser gewaschen, über Natriumsulfat getrocknet und eingedampft. Man erhält so 59,1 g (89 % d.Th.) 2,2-Dimethyl-3-[2-(4-toluolsulfonyloxy)äthyl]-chroman als gelbes Oel, das in dieser Form weiterverwendet wird.

Beispiel 2:

In eine Lösung von 5,66 g (15 mmol) N-[2-(2,2-Dimethylchroman-3-yl)äthyl]-N,N-bis(2-methoxycarbonyläthyl)-amin in 50 ml absolutem N,N-Dimethylformamid werden bei Raumtemperatur unter Rühren innert 30 Minuten 0,87 g (18 mmol) Natriumhydrid-Dispersion in Mineralöl (50 %) eingetragen. Das Reaktionsgemisch wird 1 Stunde bei Raumtemperatur gerührt und danach im Hochvakuum zur Trockne eingedampft. Der Rückstand wird mit Diäthyläther versetzt und mit kalter 2N-Salzsäure extrahiert. Die vereinigten salzsauren Extrakte werden mit Dichlormethan ausgeschüttelt und die Dichlormethan-Phasen über Natriumsulfat getrocknet und im Vakuum eingedampft. Man erhält 1,95 g (34 % d.Th.) 1-[2-(2,2-Dimethylchroman-3-yl)äthyl]-4-hydroxy-1,2,5,6-tetrahydro-pyridin-3-carbonsäuremethylester-hydrochlorid bzw. 1-[2-(2,2-Dimethylchroman-3-yl)äthyl]-4-oxo-piperidin-3-carbonsäuremethylester-hydrochlorid, das nach Umkristallisation aus Methanol/Diäthyläther einen Zersetzungsbereich von 168-170° besitzt.

N-[2-(2,2-Dimethylchroman-3-yl)äthyl]-N,N-bis(2-methoxycarbonyläthyl)amin kann z.B. auf folgende Weise hergestellt werden:

Eine Lösung von 14,4 g (40 mmol) 2,2-Dimethyl-3-[2-(4-toluolsulfonyloxy)äthyl]-chroman (hergestellt wie in Beispiel 1 beschrieben) in 200 ml Aethanol wird mit einer Lösung von 3,9 g (60 mmol) Natriumazid in 10 ml Wasser versetzt. Das Gemisch wird während 18 Stunden unter Rückfluss gekocht. Nach dem Erkalten wird das Aethanol im Vakuum abgedampft und der Rückstand mit Wasser versetzt und mit Dichlormethan extrahiert. Die Dichlormethan-Phase wird mit Wasser gewaschen, über Natriumsulfat getrocknet und eingedampft. Man erhält 8,7 g (94 % d.Th.) 3-(2-Azidoäthyl)-2,2-dimethyl-chroman in Form eines gelben Oeles.

Eine Lösung von 6,94 g (30 mmol) 3-(2-Azidoäthyl)-2,2-dimethyl-chroman in 100 ml absolutem Tetrahydrofuran wird zu einer in einer Stickstoffatmosphäre gerührten Suspension von 1,14 g (30 mmol) Lithiumaluminiumhydrid in 100 ml absolutem Diäthyläther innert 1 Stunde bei Raumtemperatur zugetropft. Nach weiteren 2 Stunden Rühren wird das Reaktionsgemisch mit 1,14 ml Wasser, 1,14 ml Natronlauge (15 %) und 3,4 ml Wasser hydrolysiert. Der entstandene Niederschlag wird abgesaugt und das Filtrat im Vakuum vollständig eingedampft. Das als Rückstand erhaltene Oel wird in 150 ml Diäthyläther gelöst und die Lösung mit 2N-Salzsäure extrahiert. Die vereinigten salzsauren Extrakte werden unter Eiskühlung mit konzentrierter Natronlauge alkalisch gestellt und mit Dichlormethan extrahiert. Die vereinigten organischen Phasen werden über Natriumsulfat getrocknet und im Vakuum eingedampft. Man erhält 5,6 g (91 % d.Th.) 3-(2-Aminoäthyl)-2,2-dimethyl-chroman als gelbes Oel, dessen Hydrochlorid bei 245-248° unter Zersetzung schmilzt.

Eine Lösung von 5,13 g (25 mmol) 3-(2-Aminoäthyl)-2,2-dimethyl-chroman in 50 ml Methanol wird bei Raumtemperatur mit 4,74 g (55 mmol) Acrylsäuremethylester versetzt. Das Gemisch wird 16 Stunden bei Raumtemperatur gerührt und anschliessend im Vakuum eingedampft. Man erhält so 9,43 g (100 % d.Th.) N-[2-(2,2-Dimethylchroman-3-yl)äthyl]-N,N-bis(2-methoxycarbonyläthyl)-amin als hellgelbes Oel, das in dieser Form weiterverwendet wird.

Beispiel 3:

Man tropft zu einer Lösung von 3,08 g (15 mmol) 3-(2-Aminoäthyl)-2,2-dimethyl-chroman (hergestellt wie in Beispiel 2 beschrieben) in 75 ml Methanol unter Eiskühlung innert 15 Minuten eine Lösung von 1,3 g (15 mmol) Acrylsäuremethylester in 25 ml Methanol zu. Während 6 Stunden wird das Reaktionsgemisch bei 0° nachgerührt und anschliessend im vakuum eingedampft. Der ölige Rückstand wird an 300 g Kieselgel (0,040-0,063 mm) mit Essigsäureäthylester als Laufmittel chromatographiert. Man erhält so 2,75 g (63 % d.Th.) N-[2-(2,2-Dimethylchroman-3-yl)äthyl]-N-(2-methoxycarbonyläthyl)-amin als hellgelbes Oel, dessen Hydrochlorid bei 132-134° schmilzt.

Beispiel 4:

Man versetzt eine Lösung von 2,91 g (10 mmol) N-[2-(2,2-Di-methylchroman-3-yl)äthyl]-N-(2-methoxycarbonyläthyl)-amin (hergestellt wie in Beispiel 3 beschrieben) in 20 ml Methanol mit 15 ml einer 5N-Lösung von Ammoniak in Methanol und lässt das Reaktionsgemisch in einem geschlossenen Gefäss während 6 Tagen bei Raumtemperatur reagieren. Anschliessend wird das Gemisch eingedampft und aus dem Rückstand mit methanolischer Salzsäure das N-(2-Aminocarbonyläthyl)-N-[2-(2,2-dimethylchroman-3-yl)äthyl]-amin-hydrochlorid hergestellt, das einen Schmelzbereich von 194-196° aufweist.

Beispiel 5:

Zu einer Lösung von 2,23 g (10 mmol) 3-(2-Aminoäthyl)-2,2-dimethyl-6-fluor-chroman in 50 ml Methanol tropft man innert 15 Minuten unter Rühren bei einer Temperatur von 0 bis 5° eine Lösung von 0,86 g (9,9 mmol) Acrylsäuremethylester in 10 ml Methanol zu. Anschliessend wird das Reaktionsgemisch 16 Stunden bei 0 bis 5° nachgerührt und sodann im Vakuum eingedampft. Der ölige Rückstand wird an 200 g Kieselgel (0,040-0,063 mm) mit Essigsäureäthylester als Laufmittel chromatographiert. Man erhält so 1,9 g (61,4 % d. Th.) N-[2-(2,2-Dimethyl-6-fluor-chroman-3-yl)äthyl]-N-(2-methoxycarbonyläthyl)-amin als hellgelbes Oel. Das hieraus mit Salzsäure in Diäthyläther hergestellte N-[2-(2,2-Dimethyl-6-fluor-chroman-3-yl)äthyl]-N-(2-methoxycarbonyläthyl)-amin-hydrochlorid kristallisiert aus Aceton/Diäthyläther und schmilzt bei 129-131°.

Das 3-(2-Aminoäthyl)-2,2-dimethyl-6-fluor-chroman lässt sich z.B. folgendermassen herstellen:

Eine Lösung von 28,0 g (0,2 mol) 5-Fluor-2-hydroxy-benzaldehyd [hergestellt nach Y. Suzuki u. H. Takahashi, Chem. Pharm. Bull. 31, 1751 (1983)] in 500 ml N,N-Dimethylformamid wird unter Rühren mit 83,1 g (0,2 mol) Kaliumcarbonat versetzt. Die resultierende dickflüssige Suspension wird mit 36,3 g (0,2 mol) 3,3-Dimethylacrylsäureäthylester versetzt. Das Reaktionsgemisch wird dann 16 Stunden bei 150° gerührt und nach Abkühlen auf Raumtemperatur im Vakuum eingedampft. Der erhaltene feste Rückstand wird mit Wasser versetzt und das wässrige Gemisch mit Diäthyläther extrahiert. Die vereinigten ätherischen Phasen werden nacheinander mit Wasser, 2N-Salzsäure, 2N-Natronlauge und nochmals Wasser gewaschen, über Natriumsulfat getrocknet und im Vakuum eingedampft. Der erhaltene ölige Rückstand wird an 1000 g Kieselgel (0,040-0,063 mm) mit Toluol als Laufmittel chromatographiert. Man erhält so 19,5 g (55 % d. Th.) 2,2-Dimethyl-6-fluor-2H-chromen als hellgelbes Oel.

Eine Lösung von 17,8 g (0,1 mol) 2,2-Dimethyl-6-fluor-2H-chromen in 300 ml Trichlormethan wird unter Rühren bei 15 bis 20° mit 40,6 g (0,2 mol) m-Chlorperbenzoesäure versetzt. Zu der erhaltenen Suspension werden bei Raumtemperatur 17,1 g (0,15 mol) Trifluoressigsäure rasch zugegeben. Das Reaktionsgemisch wird 2 Stunden bei Raumtemperatur nachgerührt und dann mit einer wässrigen Lösung von Natriumsulfit/Natriumhydrogencarbonat (1:1) versetzt. Die organische Phase wird abgetrennt, nacheinander mit Wasser und gesättigter Natriumchloridlösung gewaschen, über Natriumsulfat getrocknet und im Vakuum eingedampft. Der erhaltene Rückstand wird im Hochvakuum bei 160° der Kugelrohrdestillation unterworfen. Das Destillat wird in Diäthyläther gelöst und die Lösung nacheinander mit Natriumhydrogencarbonatlösung und gesättigter Natriumchloridlösung gewaschen, über Natriumsulfat getrocknet und erneut im Vakuum eingedampft. Der resultierende ölige Rückstand wird an 500 g Kieselgel (0,040-0,063 mm) mit Toluol als Laufmittel chromatographiert. Man erhält auf diese Weise 7,76 g (40 % d. Th.) 2,2-Dimethyl-6-fluor-3-oxo-chroman als hellgelbes Oel.

Eine Lösung von 5,7 g (29 mmol) 2,2-Dimethyl-6-fluor-3-oxo-chroman in 100 ml absolutem Tetrahydrofuran wird innert 30 Minuten unter Rühren bei 0 bis 5° zu einer Suspension von 1,69 g (35 mmol) Natriumhydrid-Dispersion (55 % in Mineralöl), die zuvor durch mehrmaliges Waschen mit n-Hexan weitgehend vom Mineralöl befreit worden ist, in 100 ml absolutem Tetrahydrofuran zugetropft. Anschliessend wird 30 Minuten bei 0 bis 5° nachgerührt und sodann eine Lösung von 7,9 g (35 mmol) Phosphonoessigsäuretriäthylester der Formel $(H_5C_2O)_2P(=O)CH_2C(=O)OC_2H_5$ in 70 ml absolutem Tetrahydrofuran innert 30 Minuten bei 0 bis 5° zugetropft. Das Reaktionsgemisch wird weitere 30 Minuten bei 0 bis 5° und dann 16 Stunden bei Raumtemperatur nachgerührt und danach in 700 ml kalte Phosphatpuffer-Lösung (pH=6) gegossen. Das resultierende Gemisch wird mit Diäthyläther extrahiert. Die vereinigten organischen Phasen werden nacheinander mit Phosphatpuffer-Lösung (pH=7) und Wasser gewaschen, über Natriumsulfat getrocknet und eingedampft. Das erhaltene rote Oel wird an 400 g Kieselgel (0,040-0,063 mm) mit Toluol als Laufmittel chromatographiert. Man erhält so 5,76 g (75 % d. Th.) 3-Aethoxycarbonylmethyl2,2-dimethyl-6-fluor-2H-chromen in Form eines orange-farbenen Oels.

Eine Lösung von 3,4 g (13 mmol) 3-Aethoxycarbonylmethyl-2,2-dimethyl-6-fluor-2H-chromen in 30 ml absolutem Aethanol wird mit 140 mg Palladium auf Kohle (5 %) versetzt und bei Normaldruck und Raumtemperatur während 90 Minuten hydriert. Der Katalysator wird sodann abfiltriert und das Filtrat im Vakuum eingedampft. Man erhält so 3,47 g 3-Aethoxycarbonylmethyl-2,2-dimethyl-6-fluor-chroman als hellgelbes Oel.

Zu einer Suspension von 0,97 g (25,5 mmol) Lithiumaluminiumhydrid in 30 ml absolutem Diäthyläther wird innert 30 Minuten unter Eiskühlung eine Lösung von 3,4 g (12,7 mmol) 3-Aethoxycarbonylmethyl-2,2-dimethyl-6-fluor-chroman in 40 ml absolutem Diäthyläther zugetropft. Das Reaktionsgemisch wird 2 Stunden bei Raumtemperatur nachgerührt und dann vorsichtig mit 1 ml Wasser, 1 ml Natronlauge (15 %) und 3 ml Wasser zersetzt. Der entstandene Niederschlag wird abgesaugt und das über Natriumsulfat getrocknete Filtrat im Vakuum eingedampft. Man erhält so 2,75 g (96 % d. Th.) 2,2-Dimethyl-6-fluor-3-(2-hydroxyäthyl)-chroman als hellgelbes Oel.

Eine Lösung von 4,48 g (20 mmol) 2,2-Dimethyl-6-fluor-3-(2-hydroxyäthyl)-chroman in 30 ml absolutem Pyridin wird bei Raumtemperatur unter Rühren mit 4,01 g (21 mmol) 4-Toluolsulfonylchlorid versetzt, wobei die leicht exotherme Reaktion mit einem Eisbad bei Raumtemperatur gehalten wird. Man rührt das Reaktionsgemisch 2 Stunden bei Raumtemperatur nach, giesst es dann auf Eiswasser und extrahiert die Mischung mit Diäthyläther. Die vereinigten organischen Phasen werden kalt mit 2N-Salzsäure und dann mit Wasser gewaschen, über Natriumsulfat getrocknet und im Vakuum eingedampft. Man erhält so 6,5 g (85,8 % d. Th.) 2,2-Dimethyl6-fluor-3-[2-(4-toluolsulfonyloxy)äthyl]-chroman als hellgelbes Oel.

Eine Lösung von 6,3 g (16,6 mmol) 2,2-Dimethyl-6-fluor-3-[2-(4-toluolsulfonyloxy)äthyl]-chroman in 100 ml Aethanol wird mit einer Lösung von 1,62 g (24,9 mmol) Natriumazid in 10 ml Wasser versetzt. Das Gemisch wird 18 Stunden unter Rückfluss gekocht. Nach dem Erkalten wird das Aethanol im Vakuum abgedampft und der Rückstand mit Wasser versetzt. Das wässrige Gemisch wird mit Diäthyläther extrahiert. Die vereinigten organischen Phasen werden mit Wasser gewaschen, über Natriumsulfat getrocknet und im Vakuum eingedampft. Man erhält so 4,13 g (100 % d. Th.) 3-(2-Azidoäthyl)-2,2-dimethyl-6-fluor-chroman als hellgelbes Oel.

Zu einer Suspension von 0,61 g (16 mmol) Lithiumaluminiumhydrid in 30 ml absolutem Diäthyläther wird innert 30 Minuten eine Lösung von 4,0 g (16 mmol) 3-(2-Azidoäthyl)-2,2-dimethyl-6-fluor-chroman in 30 ml absolutem Tetrahydrofuran zugetropft. Das Reaktionsgemisch wird 2 Stunden bei Raumtemperatur nachgerührt und dann vorsichtig mit 0,61 ml Wasser, 0,61 ml Natronlauge (15 %) und 1,83 ml Wasser zersetzt. Der entstandene Niederschlag wird abgesaugt und das Filtrat im Vakuum eingedampft. Der ölige Rückstand wird in Diäthyläther gelöst und die Lösung mit 2N-Salzsäure extrahiert. Die vereinigten salzsauren Extrakte werden unter Eiskühlung mit Natronlauge (30 %) alkalisch gestellt und mit Dichlormethan extrahiert. Die vereinigten Dichlormethan-Phasen werden über Natriumsulfat getrocknet und im Vakuum eingedampft. Man erhält so 3,21 g (90 % d. Th.) 3-(2-Aminoäthyl)-2,2-dimethyl-6-fluor-chroman als gelbes Oel. Das hieraus mit Salzsäure in Diäthyläther hergestellte 3-(2-Aminoäthyl)-2,2-dimethyl-6-fluor-chroman-hydrochlorid kristallisiert aus Methanol/Diäthyläther und besitzt einen Schmelzbereich von 249 bis 251°.

Beispiel 6:

In analoger Weise wie in den Beispielen 1 bis 5 beschrieben kann man auch die folgenden Verbindungen oder jeweils ein Salz davon herstellen:
das N-(2,2-Dimethylchroman-3-ylmethyl)-N-(2-methoxycarbonyläthyl)-amin;
das N-(2,2-Dimethylchroman-3-yl)-N-(2-methoxycarbonyläthyl)-amin;
das N-[2-(6-Cyano-2,2-dimethyl-chroman-3-yl)äthyl]-N-(2-methoxycarbonyläthyl)-amin;
das 3-[N-(2-(2,2-Dimethylchroman-3-yl)äthyl)amino]propionsäure-morpholid;
den 1-[2-(2,2-Dimethyl-6-fluor-chroman-3-yl)äthyl]-1,2,5,6-tetrahydro-pyridin-3-carbonsäuremethylester und
den 1-[2-(6-Cyano-2,2-dimethyl-chroman-3-yl)äthyl]-4-hydroxy-1,2,5,6-tetrahydro-pyridin-3-carbonsäuremethylester bzw.
den 1-[2-(6-Cyano-2,2-dimethyl-chroman-3-yl)äthyl]-4-oxo-piperidin-3-carbonsäuremethylester.

Beispiel 7:

Tabletten, enthaltend 25 mg des Wirkstoffs, z.B. N-[2-(2,2-Dimethylchroman-3-yl)äthyl]-N-(2-methoxycarbonyläthyl)-amin-hydrochlorid, können folgendermassen hergestellt werden:

| Bestandteile (für 1000 Tabletten): | |
|---|---|
| Wirkstoff | 25,0 g |
| Lactose | 100,7 g |
| Weizenstärke | 7,5 g |
| Polyäthylenglykol 6000 | 5,0 g |
| Talkum | 5,0 g |
| Magnesiumstearat | 1,8 g |
| entmineralisiertes Wasser | q.s. |

Herstellung: Sämtliche festen Ingredienzien werden zunächst durch ein Sieb mit 0,6 mm Maschenweite getrieben. Dann werden der Wirkstoff, die Lactose, das Talkum, das Magnesiumstearat und die Hälfte der Stärke vermischt. Die andere Hälfte der Stärke wird in 40 ml Wasser suspendiert und diese Suspension zu einer siedenden Lösung des Polyäthylenglykols in 100 ml Wasser hinzugegeben. Der erhaltene Stärkekleister wird zu der Hauptmenge hinzugegeben und das Gemisch, wenn nötig unter Hinzufügen von Wasser, granuliert. Das Granulat wird über Nacht bei 35° getrocknet, durch ein Sieb mit 1,2 mm Maschenweite getrieben und zu beidseitig konkaven Tabletten von etwa 6 mm Durchmesser verpresst.

Beispiel 8:

Tabletten, enthaltend 50 mg des Wirkstoffs, z.B. N-[2-(2,2-Dimethylchroman-3-yl)äthyl]-N-(2-methoxycarbonyläthyl)-amin-hydrochlorid, werden wie folgt hergestellt:

Zusammensetzung (für 10 000 Tabletten):

| | |
|---|---|
| Wirkstoff | 500,00 g |
| Lactose | 140,80 g |
| Kartoffelstärke | 274,70 g |
| Stearinsäure | 10,00 g |
| Talk | 50,00 g |
| Magnesiumstearat | 2,50 g |
| Kolloidales Siliciumdioxid | 32,00 g |
| Aethanol | q.s. |

Ein Gemisch des Wirkstoffs, der Lactose und 194,70 g Kartoffelstärke wird mit einer äthanolischen Lösung der Stearinsäure befeuchtet und durch ein Sieb granuliert. Nach dem Trocknen mischt man die restliche Kartoffelstärke, den Talk, das Magnesiumstearat und das kolloidale Siliciumdioxid zu und presst die Mischung zu Tabletten von je 0,1 g Gewicht, die gewünschtenfalls mit Teilkerben zur feineren Anpassung der Dosierung versehen sein können.

In analoger Weise können 100 mg Wirkstoff eingearbeitet werden.

Beispiel 9:

Kapseln, enthaltend 0,025 g des Wirkstoffs, z.B. N-[2-(2,2-Dimethylchroman-3-yl)äthyl]-N-(2-methoxycarbonyläthyl)-amin-hydrochlorid, können wie folgt hergestellt werden:

Zusammensetzung (für 1000 Kapseln):

| | |
|---|---|
| Wirkstoff | 25,00 g |
| Lactose | 249,00 g |
| Gelatine | 2,00 g |
| Maisstärke | 10,00 g |
| Talk | 15,00 g |
| Wasser | q.s. |

Man mischt den Wirkstoff mit der Lactose, befeuchtet die Mischung gleichmässig mit einer wässrigen Lösung der Gelatine und granuliert sie durch ein Sieb mit einer Maschenweite von 1,2 bis 1,5 mm. Das Granulat mischt man mit der getrockneten Maisstärke und dem Talk und füllt Portionen von 300 mg in Hartgelatinekapseln (Grösse 1) ab.

Beispiel 10:

In analoger Weise wie in den Beispielen 7 bis 9 beschrieben können auch pharmazeutische Präparate, als Wirkstoff enthaltend eine Verbindung der Formel I oder ein Tautomeres und/oder ein pharmazeutisch verwendbares Salz davon oder eine andere Verbindung der Formel IVc oder ein pharmazeutisch verwendbares Salz davon, beispielsweise gemäss den Beispielen 1 bis 6, hergestellt werden.

## Patentansprüche

1. Eine Verbindung der Formel

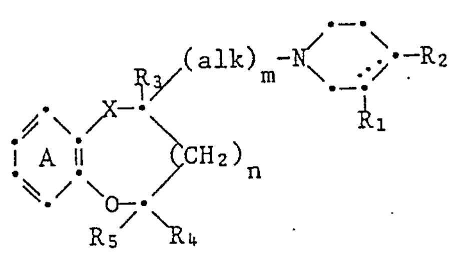

(I),

worin entweder $R_1$ Carboxy, Niederalkoxycarbonyl, amidiertes Carboxy oder gegebenenfalls acyliertes

Hydroxymethyl bedeutet und $R_2$ Wasserstoff, eine gegebenenfalls verätherte oder acylierte Hydroxygruppe oder eine gegebenenfalls acylierte Aminogruppe darstellt oder $R_1$ Wasserstoff bedeutet und $R_2$ für Carboxy, Niederalkoxycarbonyl, amidiertes Carboxy oder gegebenenfalls acyliertes Hydroxymethyl steht und worin $R_3$ Wasserstoff oder Niederalkyl bedeutet, $R_4$ Niederalkyl ist, $R_5$ Niederalkyl darstellt, alk Niederalkylen oder Niederalkyliden bedeutet, der Ring A unsubstituiert oder ein- oder mehrfach durch Hydroxy, Niederalkoxy, Niederalkanoyloxy, Cyano, Halogen, Niederalkyl und/oder Trifluormethyl substituiert ist, die gestrichelte Linie zum Ausdruck bringen soll, dass zwischen den Kohlenstoffatomen, welche die Substituenten $R_1$ und $R_2$ tragen, eine Einfach-oder eine Doppelbindung vorliegt, m 0 oder 1 bedeutet und worin entweder X ein Sauerstoffatom oder eine Methylengruppe bedeutet und n für 0 steht oder X für eine direkte Bindung steht und n für 1 steht, oder ein Tautomeres und/oder Salz davon.

2. Eine Verbindung der Formel I gemäss Anspruch 1, worin entweder $R_1$ Carboxy, Niederalkoxycarbonyl, Carbamyl, N-Niederalkylcarbamyl, N,N-Diniederalkylcarbamyl, N,N-Niederalkylencarbamyl, N,N-(Aza)-, N,N-(Oxa)- oder N,N-(Thia)niederalkylencarbamyl, Hydroxymethyl, Niederalkanoyloxymethyl, Niederalkansulfonyloxymethyl, Benzoyloxymethyl oder Pyridoyloxymethyl bedeutet und $R_2$ Wasserstoff, Hydroxy, Niederalkoxy, Benzyloxy, Niederalkanoyloxy, Niederalkansulfonyloxy, Benzoyloxy, Pyridoyloxy, Amino, Niederalkanoylamino, Niederalkansulfonylamino, Benzoylamino oder Pyridoylamino darstellt oder $R_1$ Wasserstoff bedeutet und $R_2$ für Carboxy, Niederalkoxycarbonyl, Carbamyl, N-Niederalkylcarbamyl, N,N-Diniederalkylcarbamyl, N,N-Niederalkylencarbamyl, N,N-(Aza)-, N,N-(Oxa)- oder N,N-(Thia)niederalkylencarbamyl, Hydroxymethyl, Niederalkanoyloxymethyl, Niederalkansulfonyloxymethyl, Benzoyloxymethyl oder Pyridoyloxymethyl steht und worin $R_3$ Wasserstoff oder Niederalkyl bedeutet, $R_4$ Niederalkyl ist, $R_5$ Niederalkyl darstellt, alk Niederalkylen, das die beiden in Formel I eingezeichneten Ringsysteme durch bis und mit 3 C-Atome überbrückt, oder Niederalkyliden darstellt, der Ring A unsubstituiert ist oder ein-, zwei- oder mehrfach durch Hydroxy, Niederalkoxy, Niederalkanoyloxy, Cyano, Halogen, Niederalkyl und/oder Trifluormethyl substituiert ist, die gestrichelte Linie zum Ausdruck bringen soll, dass zwischen den Kohlenstoffatomen, welche die Substituenten $R_1$ und $R_2$ tragen, eine Einfach- oder eine Doppelbindung vorliegt, m 0 oder 1 bedeutet und worin entweder X ein Sauerstoffatom oder eine Methylengruppe bedeutet und n für 0 steht oder X für eine direkte Bindung steht und n für 1 steht, oder ein Tautomeres und/oder Salz davon.

3. Eine Verbindung der Formel I gemäss Anspruch 1, worin $R_1$ $C_1$-$C_4$-Alkoxycarbonyl oder Carbamyl bedeutet, $R_2$ Wasserstoff oder Hydroxy bedeutet, $R_3$ Wasserstoff ist, $R_4$ $C_1$-$C_4$-Alkyl bedeutet, $R_5$ $C_1$-$C_4$-Alkyl darstellt, alk für $C_1$-$C_4$-Alkylen, welches die beiden in Formel I eingezeichneten Ringsysteme durch bis und mit 3 C-Atome überbrückt, steht, der Ring A unsubstituiert oder, insbesondere in 6-Stellung, durch $C_1$-$C_4$-Alkoxy, $C_1$-$C_4$-Alkyl, Halogen mit einer Atomnummer bis und mit 35, Cyano oder Trifluormethyl substituiert ist, die gestrichelte Linie zum Ausdruck bringen soll, dass zwischen den Kohlenstoffatomen, welche die Substituenten $R_1$ und $R_2$ tragen, eine Einfach- oder eine Doppelbindung vorliegt, m für 1 steht, X ein Sauerstoffatom oder eine Methylengruppe bedeutet und n für 0 steht, oder ein Tautomeres und/oder Salz davon.

4. Eine Verbindung der Formel I gemäss Anspruch 1, worin $R_1$ $C_1$-$C_4$-Alkoxycarbonyl bedeutet, $R_2$ Wasserstoff darstellt, $R_3$ Wasserstoff ist, $R_4$ $C_1$-$C_4$-Alkyl bedeutet, $R_5$ $C_1$-$C_4$-Alkyl darstellt, alk für Aethylen steht, der Ring A unsubstituiert oder, insbesondere in 6-Stellung, einfach durch Halogen mit einer Atomnummer bis und mit 35 substituiert ist, die gestrichelte Linie zum Ausdruck bringen soll, dass zwischen den Kohlen stoffatomen, welche die Substituenten $R_1$ und $R_2$ tragen, eine Doppelbindung vorliegt, m für 1 steht, X eine Methylengruppe darstellt und n für 0 steht, oder ein Salz davon.

5. Eine Verbindung der Formel I gemäss Anspruch 1, worin $R_1$ $C_1$-$C_4$-Alkoxycarbonyl bedeutet, $R_2$ Wasserstoff darstellt, $R_3$ Wasserstoff ist, $R_4$ $C_1$-$C_4$-Alkyl bedeutet, $R_5$ $C_1$-$C_4$-Alkyl darstellt, alk für Aethylen steht, der Ring A unsubstituiert ist, die gestrichelte Linie zum Ausdruck bringen soll, dass zwischen den Kohlenstoffatomen, welche die Substituenten $R_1$ und $R_2$ tragen, eine Doppelbindung vorliegt, m für 1 steht, X eine Methylengruppe darstellt und n für 0 steht, oder ein Salz davon.

6. 1-[2-(2,2-Dimethylchroman-3-yl)äthyl]-1,2,5,6-tetrahydro-pyridin-3-carbonsäuremethylester oder ein Salz davon.

7. 1-[2-(2,2-Dimethylchroman-3-yl)äthyl]-4-hydroxy-1,2,5,6-tetrahydro-pyridin-3-carbonsäuremethylester bzw. 1-[2-(2,2-Dimethylchroman-3-yl)äthyl]-4-oxo-piperidin-3-carbonsäuremethylester oder ein Salz davon.

8. 1-[2-(2,2-Dimethyl-6-fluor-chroman-3-yl)äthyl]-1,2,5,6-tetrahydro-pyridin-3-carbonsäuremethylester oder 1-[2-(6-Cyano-2,2-dimethyl-chroman-3-yl)äthyl]-4-hydroxy-1,2,5,6-tetrahydro-pyridin-3-carbonsäuremethylester bzw. 1-[2-(6-Cyano-2,2-dimethyl-chroman-3-yl)äthyl]-4-oxo-piperidin-3-carbonsäuremethylester oder jeweils ein Salz davon.

9. Verfahren zur Herstellung einer Verbindung der Formel I oder eines Tautomeren und/oder Salzes davon gemäss einem der Ansprüche 1 bis 8, dadurch gekennzeichnet, dass man

a) eine Verbindung der Formel

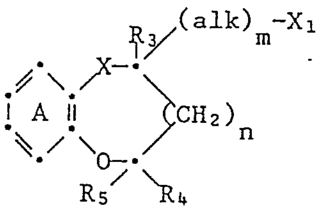

(IIa)

oder ein Salz davon, worin $X_1$ Hydroxy oder reaktionsfähiges verestertes Hydroxy bedeutet, mit einer Verbindung der Formel

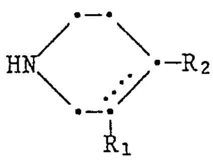

(IIb)

oder einem Tautomeren und/oder Salz davon umsetzt oder

b) in einer Verbindung der Formel

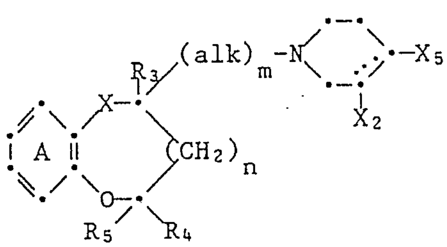

(III)

oder einem Tautomeren und/oder Salz davon, worin $X_2$ einen in von Wasserstoff verschiedenes $R_1$ überführbaren Rest bedeutet und $X_5$ einen Rest $R_a$ darstellt, wobei $R_a$ Wasserstoff, eine gegebenenfalls verätherte oder acylierte Hydroxygruppe oder eine gegebenenfalls acylierte Aminogruppe ist, $X_2$ in von Wasserstoff verschiedenes $R_1$ überführt oder in einer Verbindung der Formel III oder einem Tautomeren und/oder Salz davon, worin $X_2$ Wasserstoff ist und $X_5$ einen in $R_b$ überführbaren Rest bedeutet, wobei $R_b$ für einen von einem Rest $R_a$ verschiedenen Rest $R_2$ steht, $X_5$ in $R_b$ überführt oder

c) zur Herstellung einer Verbindung der Formel I oder eines Tautomeren und/oder Salzes davon, worin $R_2$ Hydroxy oder Amino bedeutet und worin $R_1$ von Wasserstoff und gegebenenfalls acyliertem Hydroxymethyl verschieden ist, eine Verbindung der Formel

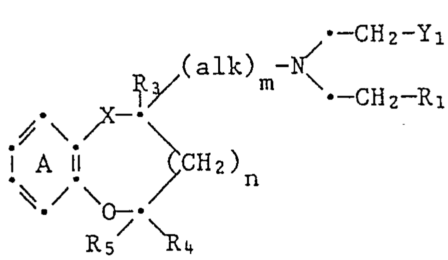

(IV),

worin $Y_1$ eine Gruppe der Formel $-CH=R_2'$ , $-C(Y_2)=R_2'$ , $-CH(Y_2)-R_2$ oder Cyano darstellt, wobei $R_2'$ Oxo oder Imino bedeutet und $Y_2$ einen abspaltbaren Rest darstellt, oder ein Salz davon cyclisiert oder

d) zur Herstellung einer Verbindung der Formel I', worin $R_2'$ Oxo oder Imino bedeutet und $R_1$ von Wasserstoff verschieden ist, oder eines Tautomeren und/oder Salzes davon eine Verbindung der

Formel

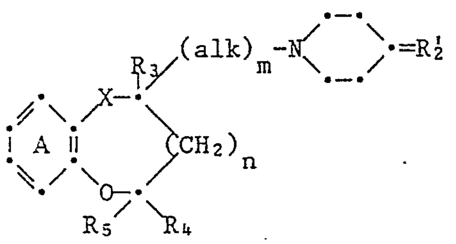

(Va)

oder ein Tautomeres und/oder ein Salz davon mit einer Verbindung der Formel

$X_3$-$R_1$ (Vb)

oder einem Salz davon, worin $R_1$ von Wasserstoff verschieden ist und $X_3$ Halogen oder Niederalkoxy bedeutet, umsetzt oder

e) zur Herstellung einer Verbindung der Formel I oder eines Tautomeren und/oder Salzes davon, worin $R_2$ eine gegebenenfalls verätherte oder acylierte Hydroxygruppe oder eine gegebenenfalls acylierte Aminogruppe ist, in einer Verbindung der Formel

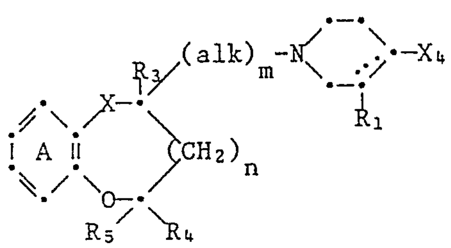

(VI)

oder einem Salz davon, worin X einen in $R_2$ überführbaren Rest bedeutet, $X_4$ in $R_2$ überführt oder

f) in einem Salz der Formel

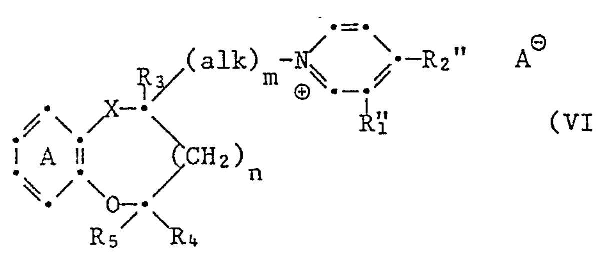

(VII),

worin $A^{\ominus}$ für das Anion einer Säure steht, $R_1''$ ein Rest $R_1$ der veräthertes oder geschütztes Hydroxymethyl ist und $R_2''$ einen Rest $R_2$, geschütztes Hydroxy, geschütztes Amino oder veräthertes oder geschütztes Hydroxymethyl bedeutet, die überschüssigen Doppelbindungen zu Einfachbindungen reduziert und, falls $R_1''$ von $R_1$ und/oder $R_2''$ von $R_2$ verschieden ist, $R_1''$ in $R_1$ und/oder $R_2''$ in $R_2$ überführt oder

g) zur Herstellung einer Verbindung der Formel I oder eines Tautomeren und/oder Salzes davon, worin $R_2$ Carboxy, amidiertes Carboxy oder Niederalkoxycarbonyl bedeutet und die gestrichelte Linie zum Ausdruck bringen soll, dass zwischen den Kohlenstoffatomen, welche die Substituenten $R_1$ und $R_2$ tragen, eine Einfachbindung vorliegt, eine Verbindung der Formel

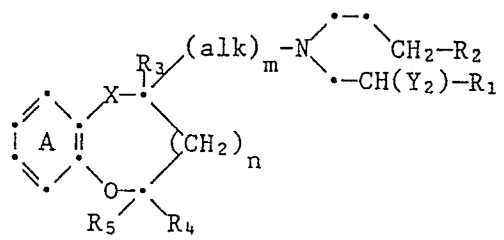

(VIII),

worin $Y_2$ einen abspaltbaren Rest darstellt, oder ein Salz davon cyclisiert und jeweils eine gegebenenfalls vorhandene Schutzgruppe abspaltet und jeweils gewünschtenfalls eine verfahrensgemäss oder auf andere Weise erhältliche Verbindung der Formel I in eine andere Verbindung der Formel I überführt, jeweils ein verfahrensgemäss erhältliches Isomerengemisch in die Komponenten auftrennt, jeweils ein verfahrensgemäss erhältliches Enantiomeren- bzw. Diastereomerengemisch in die Enantiomeren bzw. Diastereomeren aufspaltet und/oder jeweils eine verfahrensgemäss erhältliche freie Verbindung der Formel I in ein Salz überführt oder ein verfahrensgemäss erhältliches Salz in die freie Verbindung der Formel I oder in ein anderes Salz umwandelt.

10. Eine Verbindung der Formel

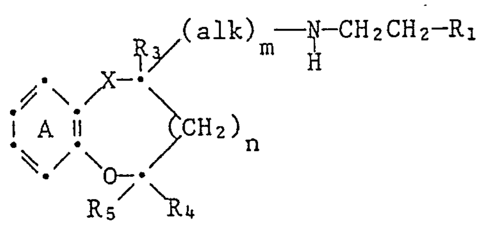

(IVc),

worin $R_1$ Carboxy, Niederalkoxycarbonyl, amidiertes Carboxy oder gegebenenfalls acyliertes Hydroxymethyl bedeutet, $R_3$ Wasserstoff oder Niederalkyl darstellt, $R_4$ Niederalkyl ist, $R_5$ Niederalkyl darstellt, alk Niederalkylen oder Niederalkyliden bedeutet, der Ring A unsubstituiert oder ein- oder mehrfach durch Hydroxy, Niederalkoxy, Niederalkanoyloxy, Cyano, Halogen, Niederalkyl und/oder Trifluormethyl substituiert ist, m 0 oder 1 bedeutet und worin entweder X ein Sauerstoffatom oder eine Methylengruppe bedeutet und n für 0 steht oder X für eine direkte Bindung steht und n für 1 steht, oder ein Salz davon.

11. Eine Verbindung der Formel IVc gemäss Anspruch 10, worin $R_1$ Carboxy, Niederalkoxycarbonyl, Carbamyl, N-Niederalkylcarbamyl, N,N-Diniederalkylcarbamyl, N,N-Niederalkylencarbamyl, N,N-(Aza)-, N,N-(Oxa)- oder N,N-(Thia)niederalkylencarbamyl, Hydroxymethyl, Niederalkanoyloxymethyl, Niederalkansulfonyloxymethyl, Benzoyloxymethyl oder Pyridoyloxymethyl bedeutet, $R_3$ Wasserstoff oder Niederalkyl darstellt, $R_4$ Niederalkyl ist, $R_5$ Niederalkyl darstellt, alk Niederalkylen, welches das Ringsystem mit der in Formel IVc eingezeichneten NH-Gruppe durch bis und mit 3 C-Atome verknüpft, oder Niederalkyliden darstellt, der Ring A unsubstituiert ist oder ein-, zwei- oder mehrfach durch Hydroxy, Niederalkoxy, Niederalkanoyloxy, Cyano, Halogen, Niederalkyl und/oder Trifluormethyl substituiert ist, m 0 oder 1 bedeutet und worin entweder X ein Sauerstoffatom oder eine Methylengruppe bedeutet und n für 0 steht oder X für eine direkte Bindung steht und n für 1 steht, oder ein Salz davon.

12. Eine Verbindung der Formel IVc gemäss Anspruch 10, worin $R_1$ $C_1$-$C_4$-Alkoxycarbonyl, Carbamyl, N,N-Niederalkylencarbamyl, N,N-(Aza)niederalkylencarbamyl, N,N-(Oxa)niederalkylencarbamyl oder N,N-(Thia)niederalkylencarbamyl bedeutet, $R_3$ Wasserstoff ist, $R_4$ $C_1$-$C_4$-Alkyl bedeutet, $R_5$ $C_1$-$C_4$-Alkyl darstellt, alk für $C_1$-$C_4$-Alkylen, welches das Ringsystem mit der in Formel IVc eingezeichneten NH-Gruppe durch bis und mit 3 C-Atome verknüpft, steht, der Ring A unsubstituiert oder, insbesondere in 6-Stellung, durch $C_1$-$C_4$-Alkoxy, $C_1$-$C_4$-Alkyl, Halogen mit einer Atomnummer bis und mit 35, Cyano oder Trifluormethyl substituiert ist, m für 0 oder 1 steht, X ein Sauerstoffatom oder eine Methylengruppe bedeutet und n für 0 steht, oder ein Salz davon.

13. Eine Verbindung der Formel IVc gemäss Anspruch 10, worin $R_1$ $C_1$-$C_4$-Alkoxycarbonyl oder Carbamyl bedeutet, $R_3$ Wasserstoff ist, $R_4$ $C_1$-$C_4$-Alkyl bedeutet, $R_5$ $C_1$-$C_4$-Alkyl darstellt, alk für $C_1$-$C_4$-Alkylen, welches das Ringsystem mit der in Formel IVc eingezeichneten NH-Gruppe durch bis und mit 3 C-Atome verknüpft, steht, der Ring A unsubstituiert oder, insbesondere in 6-Stellung, einfach durch Halogen mit einer Atomnummer bis und mit 35 substituiert ist, m für 1 steht, X eine Methylengruppe darstellt und n für 0 steht, oder ein Salz davon.

14. Eine Verbindung der Formel IVc gemäss Anspruch 10, worin $R_1$ $C_1$-$C_4$-Alkoxycarbonyl oder Carbamyl bedeutet, $R_3$ Wasserstoff ist, $R_4$ $C_1$-$C_4$-Alkyl bedeutet, $R_5$ $C_1$-$C_4$-Alkyl darstellt, alk für Aethylen steht, der Ring A unsubstituiert ist, m für 1 steht, X eine Methylengruppe darstellt und n für 0 steht, oder

ein Salz davon.

15. N-[2-(2,2-Dimethylchroman-3-yl)äthyl]-N-(2-methoxycarbonyläthyl)-amin oder ein Salz davon.

16. N-(2-Aminocarbonyläthyl)-N-[2-(2,2-dimethylchroman-3-yl)äthyl]-amin oder ein Salz davon.

17. N-[2-(2,2-Dimethyl-6-fluor-chroman-3-yl)äthyl]-N-(2-methoxycarbonyl-äthyl)-amin oder ein Salz davon.

18. N-(2,2-Dimethylchroman-3-ylmethyl)-N-(2-methoxycarbonyläthyl)-amin, N-(2,2-Dimethylchroman-3-yl)-N-(2-methoxycarbonyläthyl)-amin, N-[2-(6-Cyano-2,2-dimethyl-chroman-3-yl)äthyl]-N-(2-methoxycarbonyläthyl)-amin oder 3-[N-(2-(2,2-Dimethylchroman-3-yl)äthyl)amino]propionsäure-morpholid oder jeweils ein Salz davon.

19. Verfahren zur Herstellung einer Verbindung der Formel IVc oder eines Salzes davon gemäss einem der Ansprüche 10 bis 18, dadurch gekennzeichnet, dass man

h) Verbindungen der Formeln

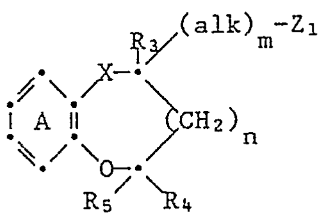

(VIIIa)

und $Z_2$-$CH_2$-$CH(Z_3)$-$R_1$ (VIIIb),

worin einer der Reste $Z_1$ und $Z_2$ reaktionsfähiges verestertes Hydroxy, der andere Amino und $Z_3$ Wasserstoff bedeutet oder $Z_1$ Amino ist und $Z_2$ sowie $Z_3$ gemeinsam eine zusätzliche Bindung darstellen, oder gegebenenfalls Salze dieser Verbindungen untereinander umsetzt oder

i) in einer Verbindung der Formel

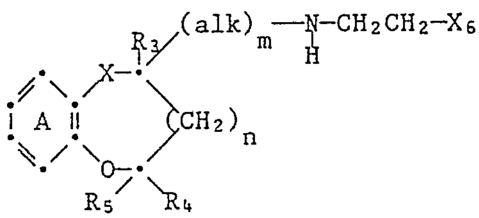

(IX),

worin $X_6$ einen in $R_1$ überführbaren Rest bedeutet, oder einem Salz davon $X_6$ in $R_1$ überführt und jeweils eine gegebenenfalls vorhandene Schutzgruppe abspaltet und jeweils gewünschtenfalls eine verfahrensgemäss oder auf andere Weise erhältliche Verbindung der Formel IVc in eine andere Verbindung der Formel IVc überführt, jeweils ein verfahrensgemäss erhältliches Isomerengemisch in die Komponenten auftrennt, jeweils ein verfahrensgemäss erhältliches Enantiomeren- bzw. Diastereomerengemisch in die Enantiomeren bzw. Diastereomeren aufspaltet und/oder jeweils eine verfahrensgemäss erhältliche freie Verbindung der Formel IVc in ein Salz überführt oder ein verfahrensgemäss erhältliches Salz in die freie Verbindung der Formel IVc oder in ein anderes Salz umwandelt.

20. Eine Verbindung gemäss einem der Ansprüche 1 bis 8 und 10 bis 18 oder gegebenenfalls ein Tautomeres und/oder ein pharmazeutisch verwendbares Salz davon zur Anwendung in einem Verfahren zur therapeutischen und/oder prophylaktischen Behandlung des menschlichen oder tierischen Körpers.

21. Eine Verbindung gemäss einem der Ansprüche 1 bis 3, 5 bis 8, 10 bis 12 und 14 bis 18 oder gegebenenfalls ein Tautomeres und/oder ein pharmazeutisch verwendbares Salz davon zur Anwendung in einem Verfahren zur therapeutischen und/oder prophylaktischen Behandlung des menschlichen oder tierischen Körpers.

22. Eine Verbindung gemäss einem der Ansprüche 1 bis 8, 10 bis 18, 20 und 21 oder gegebenenfalls ein Tautomeres und/oder ein pharmazeutisch verwendbares Salz davon zur Anwendung als Nootropikum.

23. Eine Verbindung gemäss einem der Ansprüche 1 bis 3, 5 bis 8, 10 bis 12, 14 bis 18 und 21 oder gegebenenfalls ein Tautomeres und/oder ein pharmazeutisch verwendbares Salz davon zur Anwendung als Nootropikum.

24. Ein pharmazeutisches Präparat, als Wirkstoff enthaltend eine Verbindung gemäss einem der Ansprüche 1 bis 8, 10 bis 18 und 20 bis 23 oder gegebenenfalls ein Tautomeres und/oder ein pharmazeutisch verwendbares Salz davon gegebenenfalls neben üblichen pharmazeutischen Hilfsstoffen.

25. Ein pharmazeutisches Präparat, als Wirkstoff enthaltend eine Verbindung gemäss einem der Ansprüche 1 bis 3, 5 bis 8, 10 bis 12, 14 bis 18, 21 und 23 oder gegebenenfalls ein Tautomeres und/oder ein pharmazeutisch verwendbares Salz davon gegebenenfalls neben üblichen pharmazeutischen Hilfsstoffen.

26. Ein nootropes pharmazeutisches Präparat gemäss Anspruch 24 oder 25, dadurch gekennzeichnet, dass man einen nootropen Wirkstoff wählt.

27. Ein nootropes pharmazeutisches Präparat gemäss Anspruch 25, dadurch gekennzeichnet, dass man einen nootropen Wirkstoff wählt.

28. Verwendung einer Verbindung gemäss einem der Ansprüche 1 bis 8, 10 bis 18 und 20 bis 23 oder gegebenenfalls eines Tautomeren und/oder eines pharmazeutisch verwendbaren Salzes davon zur Herstellung eines pharmazeutischen Präparats.

29. Verwendung einer Verbindung gemäss Anspruch 28 zur Herstellung eines Nootropikums.

**Patentansprüche für folgende Vertragsstaaten: ES und GR.**

1. Verfahren zur Herstellung einer Verbindung der Formel

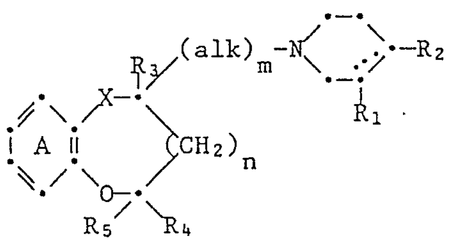

(I),

worin entweder $R_1$ Carboxy, Niederalkoxycarbonyl, amidiertes Carboxy oder gegebenenfalls acyliertes Hydroxymethyl bedeutet und $R_2$ Wasserstoff, eine gegebenenfalls verätherte oder acylierte Hydroxygruppe oder eine gegebenenfalls acylierte Aminogruppe darstellt oder $R_1$ Wasserstoff bedeutet und $R_2$ für Carboxy, Niederalkoxycarbonyl, amidiertes Carboxy oder gegebenenfalls acyliertes Hydroxymethyl steht und worin $R_3$ Wasserstoff oder Niederalkyl bedeutet, $R_4$ Niederalkyl ist, $R_5$ Niederalkyl darstellt, alk Niederalkylen oder Niederalkyliden bedeutet, der Ring A unsubstituiert oder ein- oder mehrfach durch Hydroxy, Niederalkoxy, Niederalkanoyloxy, Cyano, Halogen, Niederalkyl und/oder Trifluormethyl substituiert ist, die gestrichelte Linie zum Ausdruck bringen soll, dass zwischen den Kohlenstoffatomen, welche die Substituenten $R_1$ und $R_2$ tragen, eine Einfach-oder eine Doppelbindung vorliegt, m 0 oder 1 bedeutet und worin entweder X ein Sauerstoffatom oder eine Methylengruppe bedeutet und n für 0 steht oder X für eine direkte Bindung steht und n für 1 steht, oder eines Tautomeren und/oder Salzes davon, dadurch gekennzeichnet, dass man

a) eine Verbindung der Formel

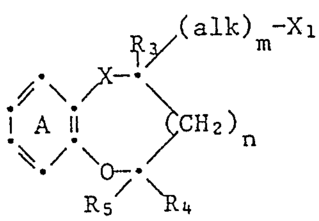

(IIa)

oder ein Salz davon, worin $X_1$ Hydroxy oder reaktionsfähiges verestertes Hydroxy bedeutet, mit einer Verbindung der Formel

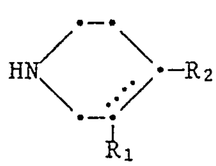

(IIb)

oder einem Tautomeren und/oder Salz davon umsetzt oder

b) in einer Verbindung der Formel

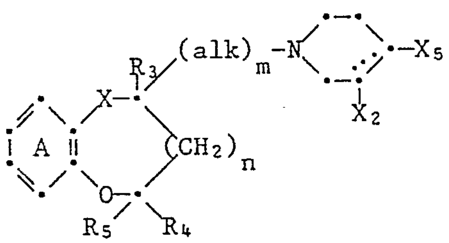

(III)

oder einem Tautomeren und/oder Salz davon, worin $X_2$ einen in von Wasserstoff verschiedenes $R_1$ überführbaren Rest bedeutet und $X_5$ einen Rest $R_a$ darstellt, wobei $R_a$ Wasserstoff, eine gegebenenfalls verätherte oder acylierte Hydroxygruppe oder eine gegebenenfalls acylierte Aminogruppe ist, $X_2$ in von Wasserstoff verschiedenes $R_1$ überführt oder in einer Verbindung der Formel III oder einem Tautomeren und/oder Salz davon, worin $X_2$ Wasserstoff ist und $X_5$ einen in $R_b$ überführbaren Rest bedeutet, wobei $R_b$ für einen von einem Rest $R_a$ verschiedenen Rest $R_2$ steht, $X_5$ in $R_b$ überführt oder

c) zur Herstellung einer Verbindung der Formel I oder eines Tautomeren und/oder Salzes davon, worin $R_2$ Hydroxy oder Amino bedeutet und worin $R_1$ von Wasserstoff und gegebenenfalls acyliertem Hydroxymethyl verschieden ist, eine Verbindung der Formel

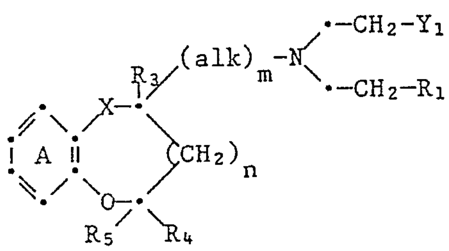

(IV),

worin $Y_1$ eine Gruppe der Formel $-CH=R_2'$, $-C(Y_2)=R_2'$, $-CH(Y_2)-R_2$ oder Cyano darstellt, wobei $R_2'$ Oxo oder Imino bedeutet und $Y_2$ einen abspaltbaren Rest darstellt, oder ein Salz davon cyclisiert oder

d) zur Herstellung einer Verbindung der Formel I', worin $R_2'$ Oxo oder Imino bedeutet und $R_1$ von Wasserstoff verschieden ist, oder eines Tautomeren und/oder Salzes davon eine Verbindung der Formel

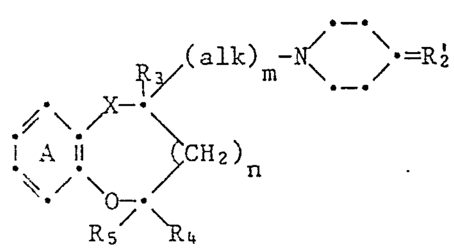

(Va)

oder ein Tautomeres und/oder ein Salz davon mit einer Verbindung der Formel

$X_3-R_1$ (Vb)

oder einem Salz davon, worin $R_1$ von Wasserstoff verschieden ist und $X_3$ Halogen oder Niederalkoxy bedeutet, umsetzt oder

e) zur Herstellung einer Verbindung der Formel I oder eines Tautomeren und/oder Salzes davon, worin $R_2$ eine gegebenenfalls verätherte oder acylierte Hydroxygruppe oder eine gegebenenfalls

acylierte Aminogruppe ist, in einer Verbindung der Formel

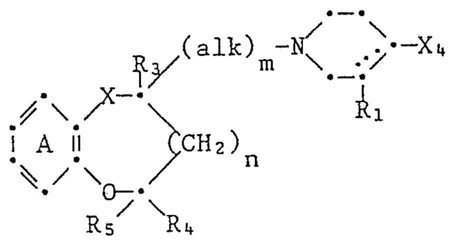

(VI)

oder einem Salz davon, worin $X_4$ einen in $R_2$ überführbaren Rest bedeutet, $X_4$ in $R_2$ überführt oder

f) in einem Salz der Formel

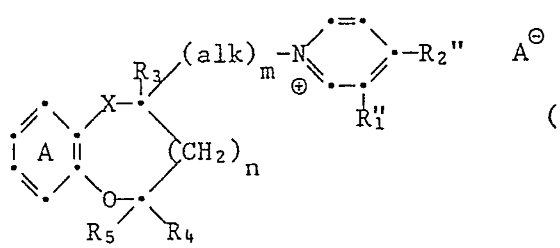

(VII),

worin $A^{\ominus}$ für das Anion einer Säure steht, $R_1''$ ein Rest $R_1$ oder veräthertes oder geschütztes Hydroxymethyl ist und $R_2''$ einen Rest $R_2$, geschütztes Hydroxy, geschütztes Amino oder veräthertes oder geschütztes Hydroxymethyl bedeutet, die überschüssigen Doppelbindungen zu Einfachbindungen reduziert und, falls $R_1''$ von $R_1$ und/oder $R_2''$ von $R_2$ verschieden ist, $R_1''$ in $R_1$ und/oder $R_2''$ in $R_2$ überführt oder

g) zur Herstellung einer Verbindung der Formel I oder eines Tautomeren und/oder Salzes davon, worin $R_2$ Carboxy, amidiertes Carboxy oder Niederalkoxycarbonyl bedeutet und die gestrichelte Linie zum Ausdruck bringen soll, dass zwischen den Kohlenstoffatomen, welche die Substituenten $R_1$ und $R_2$ tragen, eine Einfachbindung vorliegt, eine Verbindung der Formel

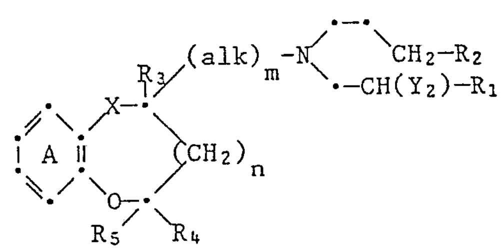

(VIII),

worin $Y_2$ einen abspaltbaren Rest darstellt, oder ein Salz davon cyclisiert und jeweils eine gegebenenfalls vorhandene Schutzgruppe abspaltet und jeweils gewünschtenfalls eine verfahrensgemäss oder auf andere Weise erhältliche Verbindung der Formel I in eine andere Verbindung der Formel I überführt, jeweils ein verfahrensgemäss erhältliches Isomerengemisch in die Komponenten auftrennt, jeweils ein verfahrensgemäss erhältliches Enantiomeren- bzw. Diastereomerengemisch in die Enantiomeren bzw. Diastereomeren aufspaltet und/oder jeweils eine verfahrensgemäss erhältliche freie Verbindung der Formel I in ein Salz überführt oder ein verfahrensgemäss erhältliches Salz in die freie Verbindung der Formel I oder in ein anderes Salz umwandelt.

2. Verfahren gemäss Anspruch 1 zur Herstellung einer Verbindung der Formel I, worin entweder $R_1$ Carboxy, Niederalkoxycarbonyl, Carbamyl, N-Niederalkylcarbamyl, N,N-Diniederalkylcarbamyl, N,N-Niederalkylencarbamyl, N,N-(Aza)-, N,N-(Oxa)- oder N,N-(Thia)niederalkylencarbamyl, Hydroxymethyl, Niederalkanoyloxymethyl, Niederalkansulfonyloxymethyl, Benzoyloxymethyl oder Pyridoyloxymethyl bedeutet und $R_2$ Wasserstoff, Hydroxy, Niederalkoxy, Benzyloxy, Niederalkanoyloxy, Niederalkansulfonyl oxy, Benzoyloxy, Pyridoyloxy, Amino, Niederalkanoylamino, Niederalkansulfonylamino, Benzoylamino

oder Pyridoylamino darstellt oder $R_1$ Wasserstoff bedeutet und $R_2$ für Carboxy, Niederalkoxycarbonyl, Carbamyl, N-Niederalkylcarbamyl, N,N-Diniederalkylcarbamyl, N,N-Niederalkylencarbamyl, N,N-(Aza)-, N,N-(Oxa)- oder N,N-(Thia)niederalkylencarbamyl, Hydroxymethyl, Niederalkanoyloxymethyl, Niederalkansulfonyloxymethyl, Benzoyloxymethyl oder Pyridoyloxymethyl steht und worin $R_3$ Wasserstoff oder Niederalkyl bedeutet, $R_4$ Niederalkyl ist, $R_5$ Niederalkyl darstellt, alk Niederalkylen, das die beiden in Formel I eingezeichneten Ringsysteme durch bis und mit 3 C-Atome überbrückt, oder Niederalkyliden darstellt, der Ring A unsubstituiert ist oder ein-, zwei- oder mehrfach durch Hydroxy, Niederalkoxy, Niederalkanoyloxy, Cyano, Halogen, Niederalkyl und/oder Trifluormethyl substituiert ist, die gestrichelte Linie zum Ausdruck bringen soll, dass zwischen den Kohlenstoffatomen, welche die Substituenten $R_1$ und $R_2$ tragen, eine Einfach- oder eine Doppelbindung vorliegt, m 0 oder 1 bedeutet und worin entweder X ein Sauerstoffatom oder eine Methylengruppe bedeutet und n für 0 steht oder X für eine direkte Bindung steht und n für 1 steht, oder eines Tautomeren und/oder Salzes davon.

3. Verfahren gemäss Anspruch 1 zur Herstellung einer Verbindung der Formel I, worin $R_1$ $C_1$-$C_4$-Alkoxycarbonyl oder Carbamyl bedeutet, $R_2$ Wasserstoff oder Hydroxy bedeutet, $R_3$ Wasserstoff ist, $R_4$ $C_1$-$C_4$-Alkyl bedeutet, $R_5$ $C_1$-$C_4$-Alkyl darstellt, alk für $C_1$-$C_4$-Alkylen, welches die beiden in Formel I eingezeichneten Ringsysteme durch bis und mit 3 C-Atome überbrückt, steht, der Ring A unsubstituiert oder, insbesondere in 6-Stellung, durch $C_1$-$C_4$-Alkoxy, $C_1$-$C_4$-Alkyl, Halogen mit einer Atomnummer bis und mit 35, Cyano oder Trifluormethyl substituiert ist, die gestrichelte Linie zum Ausdruck bringen soll, dass zwischen den Kohlenstoffatomen, welche die Substituenten $R_1$ und $R_2$ tragen, eine Einfach- oder eine Doppelbindung vorliegt, m für 1 steht, X ein Sauerstoffatom oder eine Methylengruppe bedeutet, und n für 0 steht, oder eines Tautomeren und/oder Salzes davon.

4. Verfahren gemäss Anspruch 1 zur Herstellung einer Verbindung der Formel I, worin $R_1$ $C_1$-$C_4$-Alkoxycarbonyl bedeutet, $R_2$ Wasserstoff darstellt, $R_3$ Wasserstoff ist, $R_4$ $C_1$-$C_4$-Alkyl bedeutet, $R_5$ $C_1$-$C_4$-Alkyl dar stellt, alk für Aethylen steht, der Ring A unsubstituiert oder, insbesondere in 6-Stellung, einfach durch Halogen mit einer Atomnummer bis und mit 35 substituiert ist, die gestrichelte Linie zum Ausdruck bringen soll, dass zwischen den Kohlenstoffatomen, welche die Substituenten $R_1$ und $R_2$ tragen, eine Doppelbindung vorliegt, m für 1 steht, X eine Methylengruppe darstellt und n für 0 steht, oder eines Salzes davon.

5. Verfahren gemäss Anspruch 1 zur Herstellung einer Verbindung der Formel I, worin $R_1$ $C_1$-$C_4$-Alkoxycarbonyl bedeutet, $R_2$ Wasserstoff darstellt, $R_3$ Wasserstoff ist, $R_4$ $C_1$-$C_4$-Alkyl bedeutet, $R_5$ $C_1$-$C_4$-Alkyl darstellt, alk für Aethylen steht, der Ring A unsubstituiert ist, die gestrichelte Linie zum Ausdruck bringen soll, dass zwischen den Kohlenstoffatomen, welche die Substituenten $R_1$ und $R_2$ tragen, eine Doppelbindung vorliegt, m für 1 steht, X eine Methylengruppe darstellt und n für 0 steht, oder eines Salzes davon.

6. Verfahren gemäss Anspruch 1 zur Herstellung von 1-[2-(2,2-Dimethylchroman-3-yl)äthyl]-1,2,5,6-tetrahydro-pyridin-3-carbonsäuremethylester oder eines Salzes davon.

7. Verfahren gemäss Anspruch 1 zur Herstellung von 1-[2-(2,2-Dimethylchroman-3-yl)äthyl]-4-hydroxy-1,2,5,6-tetrahydro-pyridin-3-carbonsäuremethylester bzw. 1-[2-(2,2-Dimethylchroman-3-yl)äthyl]-4-oxo-piperidin-3-carbonsäuremethylester oder eines Salzes davon.

8. Verfahren gemäss Anspruch 1 zur Herstellung von 1-[2-(2,2-Dimethyl-6-fluor-chroman--3-yl)äthyl]-1,2,5,6-tetrahydro-pyridin-3-carbonsäuremethylester oder 1-[2-(6-Cyano-2,2-dimethyl-chroman-3-yl)äthyl]-4-hydroxy-1,2,5,6-tetrahydro-pyridin-3-carbonsäuremethylester bzw. 1-[2-(6-Cyano-2,2-dimethyl-chroman-3-yl)äthyl]-4-oxo-piperidin-3-carbonsäuremethylester oder jeweils eines Salzes davon.

9. Verfahren zur Herstellung einer Verbindung der Formel

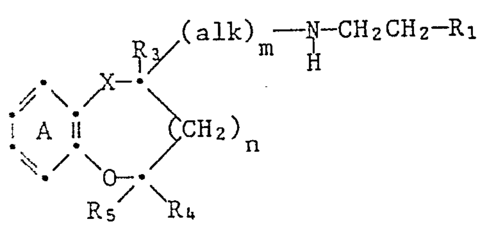

(IVc),

worin $R_1$ Carboxy, Niederalkoxycarbonyl, amidiertes Carboxy oder gegebenenfalls acyliertes Hydroxymethyl bedeutet, $R_3$ Wasserstoff oder Niederalkyl darstellt, $R_4$ Niederalkyl ist, $R_5$ Niederalkyl darstellt, alk Niederalkylen oder Niederalkyliden bedeutet, der Ring A unsubstituiert oder ein- oder mehrfach durch Hydroxy, Niederalkoxy, Niederalkanoyloxy, Cyano, Halogen, Niederalkyl und/oder Trifluormethyl substituiert ist, m 0 oder 1 bedeutet und worin entweder X ein Sauerstoffatom oder eine Methylengruppe bedeutet und n für 0 steht oder X für eine direkte Bindung steht und n für 1 steht, oder eines Salzes davon, dadurch gekennzeichnet, dass man

h) Verbindungen der Formeln

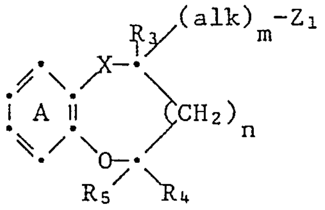

(VIIIa)

und $Z_2-CH_2-CH(Z_3)-R_1$ (VIIIb),

worin einer der Reste $Z_1$ und $Z_2$ reaktionsfähiges verestertes Hydroxy, der andere Amino und $Z_3$ Wasserstoff bedeutet oder $Z_1$ Amino ist und $Z_2$ sowie $Z_3$ gemeinsam eine zusätzliche Bindung darstellen, oder gegebenenfalls Salze dieser Verbindungen untereinander umsetzt oder

i) in einer Verbindung der Formel

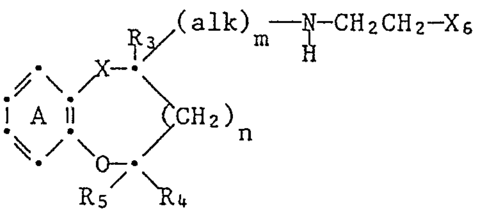

(IX),

worin $X_6$ einen in $R_1$ überführbaren Rest bedeutet, oder einem Salz davon $X_6$ in $R_1$ überführt und jeweils eine gegebenenfalls vorhandene Schutzgruppe abspaltet und jeweils gewünschtenfalls eine verfahrensgemäss oder auf andere Weise erhältliche Verbindung der Formel IVc in eine andere Verbindung der Formel IVc überführt, jeweils ein verfahrensgemäss erhältliches Isomerengemisch in die Komponenten auftrennt, jeweils ein verfahrensgemäss erhältliches Enantiomeren- bzw. Diastereomerengemisch in die Enantiomeren bzw. Diastereomeren aufspaltet und/oder jeweils eine verfahrensgemäss erhältliche freie Verbindung der Formel IVc in ein Salz überführt oder ein verfahrensgemäss erhältliches Salz in die freie Verbindung der Formel IVc oder in ein anderes Salz umwandelt.

10. Verfahren gemäss Anspruch 9 zur Herstellung einer Verbindung der Formel IVc, worin $R_1$ Carboxy, Niederalkoxycarbonyl, Carbamyl, N-Niederalkylcarbamyl, N,N-Diniederalkylcarbamyl, N,N-Niederalkylencarbamyl, N,N-(Aza)-, N,N-(Oxa)- oder N,N-(Thia)niederalkylencarbamyl, Hydroxymethyl, Niederalkanoyloxymethyl, Niederalkansulfonyloxymethyl, Benzoyloxymethyl oder Pyridoyloxymethyl bedeutet, $R_3$ Wasserstoff oder Niederalkyl darstellt, $R_4$ Niederalkyl ist, $R_5$ Niederalkyl darstellt, alk Niederalkylen, welches das Ringsystem mit der in Formel IVc eingezeichneten NH-Gruppe durch bis und mit 3 C-Atome verknüpft, oder Niederalkyliden darstellt, der Ring A unsubstituiert ist oder ein-, zwei-oder mehrfach durch Hydroxy, Niederalkoxy, Niederalkanoyloxy, Cyano, Halogen, Niederalkyl und/oder Trifluormethyl substituiert ist, m 0 oder 1 bedeutet und worin entweder X ein Sauerstoffatom oder eine Methylengruppe bedeutet und n für 0 steht oder X für eine direkte Bindung steht und n für 1 steht, oder eines Salzes davon.

11. Verfahren gemäss Anspruch 9 zur Herstellung einer Verbindung der Formel IVc, worin $R_1$ $C_1$-$C_4$-Alkoxycarbonyl, Carbamyl, N,N-Niederalkylencarbamyl, N,N-(Aza)niederalkylencarbamyl, N,N-(Oxa)niederalkylencarbamyl oder N,N-(Thia)niederalkylencarbamyl bedeutet, $R_3$ Wasserstoff ist, $R_4$ $C_1$-$C_4$-Alkyl bedeutet, $R_5$ $C_1$-$C_4$-Alkyl darstellt, alk für $C_1$-$C_4$-Alkylen, welches das Ringsystem mit der in Formel IVc eingezeichneten NH-Gruppe durch bis und mit 3 C-Atome verknüpft, steht, der Ring A unsubstituiert oder, insbesondere in 6-Stellung, durch $C_1$-$C_4$-Alkoxy, $C_1$-$C_4$-Alkyl, Halogen mit einer Atomnummer bis und mit 35, Cyano oder Trifluormethyl substituiert ist, m für 0 oder 1 steht, X ein Sauerstoffatom oder eine Methylengruppe bedeutet und n für 0 steht, oder eines Salzes davon.

12. Verfahren gemäss Anspruch 9 zur Herstellung einer Verbindung der Formel Ivc, worin $R_1$ $C_1$-$C_4$-Alkoxycarbonyl oder Carbamyl bedeutet, $R_3$ Wasserstoff ist, $R_4$ $C_1$-$C_4$-Alkyl bedeutet, $R_5$ $C_1$-$C_4$-Alkyl darstellt, alk für $C_1$-$C_4$-Alkylen, welches das Ringsystem mit der in Formel IVc eingezeichneten NH-Gruppe durch bis und mit 3 C-Atome verknüpft, steht, der Ring A unsubstituiert oder, insbesondere in 6-Stellung, einfach durch Halogen mit einer Atomnummer bis und mit 35 substituiert ist, m für 1 steht, X eine Methylengruppe darstellt und n für 0 steht, oder eines Salzes davon.

13. Verfahren gemäss Anspruch 9 zur Herstellung einer Verbindung der Formel IVc, worin $R_1$ $C_1$-$C_4$-Alkoxycarbonyl oder Carbamyl bedeutet, $R_3$ Wasserstoff ist, $R_4$ $C_1$-$C_4$-Alkyl bedeutet, $R_5$ $C_1$-$C_4$-Alkyl darstellt, alk für Aethylen steht, der Ring A unsubstituiert ist, m für 1 steht, X eine Methylengruppe darstellt und n für 0 steht, oder eines Salzes davon.

14. Verfahren gemäss Anspruch 9 zur Herstellung von N-[2-(2,2-Dimethylchroman-3-yl)äthyl]-N-(2-methoxycarbonyläthyl)-amin oder eines Salzes davon.

15. Verfahren gemäss Anspruch 9 zur Herstellung von N-(2-Aminocarbonyläthyl)-N-[2-(2,2-dimethylchroman-3-yl)äthyl]-amin oder eines Salzes davon.

16. Verfahren gemäss Anspruch 9 zur Herstellung von N-[2-(2,2-Dimethyl-6-fluor-chroman-3-yl)äthyl]-N-(2-methoxycarbonyläthyl)-amin oder eines Salzes davon.

17. Verfahren gemäss Anspruch 9 zur Herstellung von N-(2,2-Dimethylchroman-3-ylmethyl)-N-(2-methoxycarbonyläthyl)-amin, N-(2,2-Dimethylchroman-3-yl)-N-(2-methoxycarbonyläthyl)-amin, N-[2-(6-Cyano-2,2-dimethyl-chroman-3-yl)äthyl]-N-(2-methoxycarbonyläthyl)-amin oder 3-[N-(2-(2,2-Dimethylchroman-3-yl)äthyl)amino]propionsäure-morpholid oder jeweils eines Salzes davon.

18. Verfahren zur Herstellung eines pharmazeutischen Präparates, dadurch gekennzeichnet, dass man

a) eine Verbindung der Formel

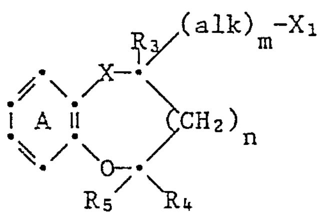

(IIa)

oder ein Salz davon, worin $X_1$ Hydroxy oder reaktionsfähiges verestertes Hydroxy bedeutet, mit einer Verbindung der Formel

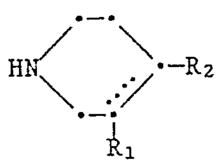

(IIb)

oder einem Tautomeren und/oder Salz davon umsetzt oder

b) in einer Verbindung der Formel

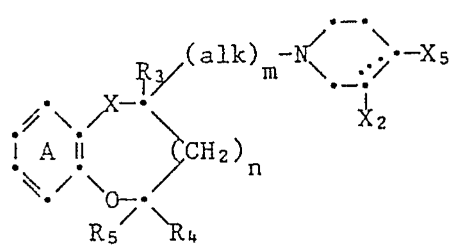

(III)

oder einem Tautomeren und/oder Salz davon, worin $X_2$ einen in von Wasserstoff verschiedenes $R_1$ überführbaren Rest bedeutet und $X_5$ einen Rest $R_a$ darstellt, wobei $R_a$ Wasserstoff, eine gegebenenfalls verätherte oder acylierte Hydroxygruppe oder eine gegebenenfalls acylierte Aminogruppe ist, $X_2$ in von Wasserstoff verschiedenes $R_1$ überführt oder in einer Verbindung der Formel III oder einem Tautomeren und/oder Salz davon, worin $X_2$ Wasserstoff ist und $X_5$ einen in $R_b$ überführbaren Rest bedeutet, wobei $R_b$ für einen von einem Rest $R_a$ verschiedenen Rest $R_2$ steht, $X_5$ in $R_b$ überführt oder

c) zur Herstellung einer Verbindung der Formel I oder eines Tautomeren und/oder Salzes davon, worin $R_2$ Hydroxy oder Amino bedeutet und worin $R_1$ von Wasserstoff und gegebenenfalls acyliertem Hydroxymethyl verschieden ist, eine Verbindung der Formel

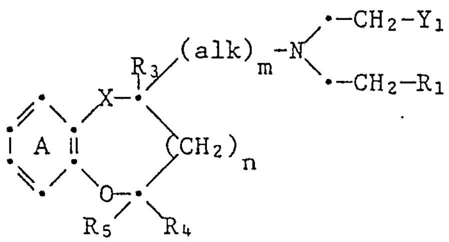

(IV),

worin $Y_1$ eine Gruppe der Formel $-CH=R_2'$ $-C(Y_2)=R_2'$ , $-CH(Y_2)-R_2$ oder Cyano darstellt, wobei $R_2'$ Oxo oder Imino bedeutet und $Y_2$ einen abspaltbaren Rest darstellt, oder ein Salz davon cyclisiert oder

d) zur Herstellung einer Verbindung der Formel I′, worin $R_2'$ Oxo oder Imino bedeutet und $R_1$ von Wasserstoff verschieden ist, oder eines Tautomeren und/oder Salzes davon eine Verbindung der Formel

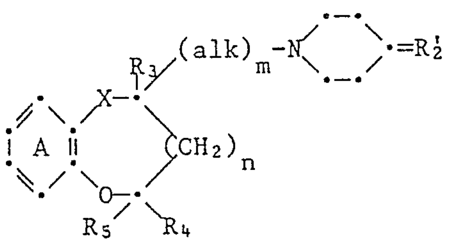

(Va)

oder ein Tautomeres und/oder ein Salz davon mit einer Verbindung der Formel

$X_3-R_1$ (Vb)

oder einem Salz davon, worin $R_1$ von Wasserstoff verschieden ist und $X_3$ Halogen oder Niederalkoxy bedeutet, umsetzt oder

e) zur Herstellung einer Verbindung der Formel I oder eines Tautomeren und/oder Salzes davon, worin $R_2$ eine gegebenenfalls verätherte oder acylierte Hydroxygruppe oder eine gegebenenfalls acylierte Aminogruppe ist, in einer Verbindung der Formel

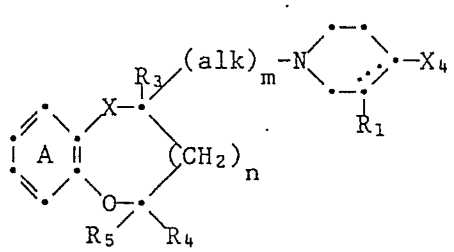

(VI)

oder einem Salz davon, worin $X_4$ einen in $R_2$ überführbaren Rest bedeutet, $X_4$ in $R_2$ überführt oder

f) in einem Salz der Formel

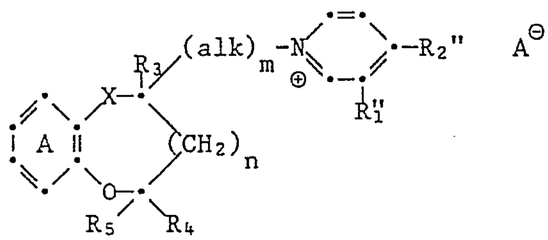

(VII),

worin $A^{\ominus}$ für das Anion einer Säure steht, $R_1''$ ein Rest $R_1$ oder veräthertes oder geschütztes Hydroxymethyl ist und $R_2''$ einen Rest $R_2$, geschütztes Hydroxy, geschütztes Amino oder veräthertes oder geschütztes Hydroxymethyl bedeutet, die überschüssigen Doppelbindungen zu Einfachbindungen reduziert und, falls $R_1''$ von $R_1$ und/oder $R_2''$ von $R_2$ verschieden ist, $R_1''$ in $R_1$ und/oder $R_2''$ in $R_2$ überführt oder

g) zur Herstellung einer Verbindung der Formel I oder eines Tautomeren und/oder Salzes davon, worin $R_2$ Carboxy, amidiertes Carboxy oder Niederalkoxycarbonyl bedeutet und die gestrichelte Linie zum Ausdruck bringen soll, dass zwischen den Kohlenstoffatomen, welche die Substituenten $R_1$ und $R_2$ tragen, eine Einfachbindung vorliegt, eine Verbindung der Formel

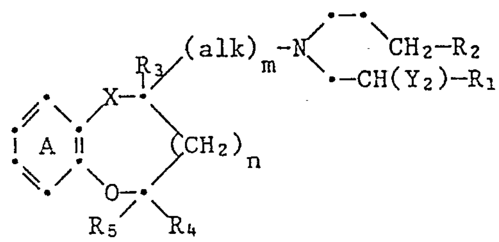

(VIII),

worin $Y_2$ einen abspaltbaren Rest darstellt, oder ein Salz davon cyclisiert und jeweils eine gegebenenfalls vorhandene Schutzgruppe abspaltet und jeweils gewünschtenfalls eine verfahrensgemäss oder auf andere Weise erhältliche Verbindung der Formel I in eine andere Verbindung der Formel I überführt, jeweils ein verfahrensgemäss erhältliches Isomerengemisch in die Komponenten auftrennt, jeweils ein verfahrensgemäss erhältliches Enantiomeren- bzw. Diastereomerengemisch in die Enantiomeren bzw. Diastereomeren aufspaltet und/oder jeweils eine verfahrensgemäss erhältliche freie Verbindung der Formel I in ein Salz überführt oder ein verfahrensgemäss erhältliches Salz in die freie Verbindung der Formel I oder in ein anderes Salz umwandelt und eine auf diese Weise jeweils erhältliche Verbindung der Formel

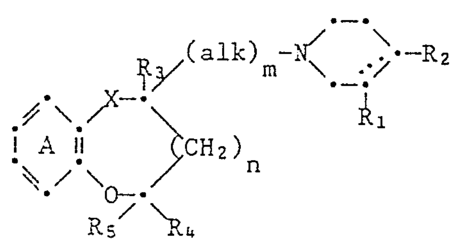

(I),

worin entweder $R_1$ Carboxy, Niederalkoxycarbonyl, amidiertes Carboxy oder gegebenenfalls acyliertes Hydroxymethyl bedeutet und $R_2$ Wasserstoff, eine gegebenenfalls verätherte oder acylierte Hydroxygruppe oder eine gegebenenfalls acylierte Aminogruppe darstellt oder $R_1$ Wasserstoff bedeutet und $R_2$ für Carboxy, Niederalkoxycarbonyl, amidiertes Carboxy oder gegebenenfalls acyliertes Hydroxymethyl steht und worin $R_3$ Wasserstoff oder Niederalkyl bedeutet, $R_4$ Niederalkyl ist, $R_5$ Niederalkyl darstellt, alk Niederalkylen oder Niederalkyliden bedeutet, der Ring A unsubstituiert oder ein- oder mehrfach durch Hydroxy, Niederalkoxy, Niederalkanoyloxy, Cyano, Halogen, Niederalkyl und/oder Trifluormethyl substituiert ist, die gestrichelte Linie zum Ausdruck bringen soll, dass zwischen den Kohlenstoffatomen, welche die Substituenten $R_1$ und $R_2$ tragen,

eine Einfach-oder eine Doppelbindung vorliegt, m 0 oder 1 bedeutet und worin entweder X ein Sauerstoffatom oder eine Methylengruppe bedeutet und n für 0 steht oder X für eine direkte Bindung steht und n für 1 steht, oder ein jeweils erhältliches Tautomeres und/oder pharmazeutisch verwendbares Salz davon, gegebenenfalls unter Beimischung von üblichen pharmazeutischen Hilfsstoffen, zu einem pharmazeutischen Präparat verarbeitet.

19. Verfahren zur Herstellung eines pharmazeutischen Präparates, dadurch gekennzeichnet, dass man

h) Verbindungen der Formeln

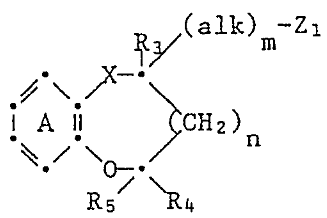

(VIIIa)

und $Z_2$-$CH_2$-$CH(Z_3)$-$R_1$ (VIIIb),

worin einer der Reste $Z_1$ und $Z_2$ reaktionsfähiges verestertes Hydroxy, der andere Amino und $Z_3$ Wasserstoff bedeutet oder $Z_1$ Amino ist und $Z_2$ sowie $Z_3$ gemeinsam eine zusätzliche Bindung darstellen, oder gegebenenfalls Salze dieser Verbindungen untereinander umsetzt oder

i) in einer Verbindung der Formel

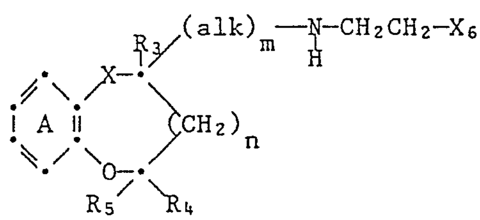

(IX),

worin $X_6$ einen in $R_1$ überführbaren Rest bedeutet, oder einem Salz davon $X_6$ in $R_1$ überführt und jeweils eine gegebenenfalls vorhandene Schutzgruppe abspaltet und jeweils gewünschtenfalls eine verfahrensgemäss oder auf andere Weise erhältliche Verbindung der Formel IVc in eine andere Verbindung der Formel IVc überführt, jeweils ein verfahrensgemäss erhältliches Isomerengemisch in die Komponenten auftrennt, jeweils ein verfahrensgemäss erhältliches Enantiomeren- bzw. Diastereomerengemisch in die Enantiomeren bzw. Diastereomeren aufspaltet und/oder jeweils eine verfahrensgemäss erhältliche freie Verbindung der Formel IVc in ein Salz überführt oder ein verfahrensgemäss erhältliches Salz in die freie Verbindung der Formel IVc oder in ein anderes Salz umwandelt und eine auf diese Weise jeweils erhältliche Verbindung der Formel

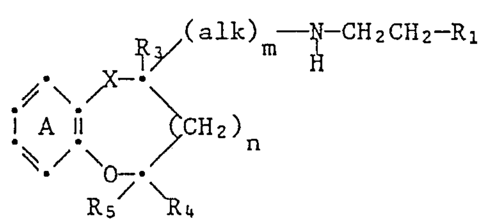

(IVc),

worin $R_1$ Carboxy, Niederalkoxycarbonyl, amidiertes Carboxy oder gegebenenfalls acyliertes Hydroxymethyl bedeutet, $R_3$ Wasserstoff oder Niederalkyl darstellt, $R_4$ Niederalkyl ist, $R_5$ Niederalkyl darstellt, alk Niederalkylen oder Niederalkyliden bedeutet, der Ring A unsubstituiert oder ein- oder mehrfach durch Hydroxy, Niederalkoxy, Niederalkanoyloxy, Cyano, Halogen, Niederalkyl und/oder Trifluormethyl substituiert ist, m 0 oder 1 bedeutet und worin entweder X ein Sauerstoffatom oder eine Methylengruppe bedeutet und n für 0 steht oder X für eine direkte Bindung steht und n für 1 steht, oder ein jeweils erhältliches pharmazeutisch verwendbares Salz davon, gegebenenfalls unter Beimischung von üblichen pharmazeutischen Hilfsstoffen, zu einem pharmazeutischen Präparat verarbeitet.

20. Verfahren zur Herstellung eines pharmazeutischen Präparates, dadurch gekennzeichnet, dass man

eine Verbindung, erhältlich gemäss einem der Ansprüche 1 bis 17, oder gegebenenfalls ein Tautomeres und/oder ein pharmazeutisch verwendbares Salz davon, gegebenenfalls unter Beimischung von üblichen pharmazeutischen Hilfsstoffen, zu einem pharmazeutischen Präparat verarbeitet.

21. Verfahren zur Herstellung eines pharmazeutischen Präparates, dadurch gekennzeichnet, dass man eine Verbindung, erhältlich gemäss einem der Ansprüche 1 bis 3, 5 bis 11 und 13 bis 17, oder gegebenenfalls ein Tautomeres und/oder ein pharmazeutisch verwendbares Salz davon, gegebenenfalls unter Beimischung von üblichen pharmazeutischen Hilfsstoffen, zu einem pharmazeutischen Präparat verarbeitet.

22. Verfahren zur Herstellung eines nootropen pharmazeutischen Präparates gemäss einem der Ansprüche 18 bis 21, dadurch gekennzeichnet, dass man einen nootropen Wirkstoff wählt.

23. Verfahren zur Herstellung eines nootropen pharmazeutischen Präparates gemäss einem der Ansprüche 18, 19 und 21, dadurch gekennzeichnet, dass man einen nootropen Wirkstoff wählt.

24. Verwendung einer Verbindung, erhältlich gemäss einem der Ansprüche 1 bis 17, oder gegebenenfalls eines Tautomeren und/oder eines pharmazeutisch verwendbaren Salzes davon zur Herstellung eines pharmazeutischen Präparats.

25. Verwendung einer Verbindung gemäss Anspruch 24 zur Herstellung eines Nootropikums.